# FASCICULE DE BREVET EUROPEEN

(11) **EP 2 477 990 B1**
(45) Date de publication et mention de la délivrance du brevet: **20.08.2014**
(21) Numéro de dépôt: 10771159.0
(22) Date de dépôt: 17.09.2010
(51) Int. Cl.: C07D 471/06, A61K 31/437

(54) **DÉRIVÉS DE 5-PHENYL-PYRAZOLOPYRIDINE, LEUR PRÉPARATION ET LEUR APPLICATION EN THÉRAPEUTIQUE**
5-PHENYL-PYRAZOLOPYRIDIN-DERIVATE, IHRE HERSTELLUNG UND THERAPEUTISCHE ANWENDUNG
5-PHENYL PYRAZOLOPYRIDINE DERIVATIVES, PREPARATION AND THERAPEUTIC USE THEREOF

(30) Priorité: 18.09.2009 FR 0956444
(43) Date de publication de la demande: 25.07.2012
(73) Titulaire: SANOFI, 75008 Paris (FR)
(72) Inventeur: AUGER, Florian, F-75013 Paris (FR); DE PERETTI, Danielle, F-75013 Paris (FR); EVEN, Luc, F-75013 Paris (FR)
(74) Mandataire: Le Coupanec, Pascale A.M.P.
(86) Numéro de dépôt international: PCT/FR2010/051932
(87) Numéro de publication internationale: WO 2011/033230

(56) Documents cités:
- WO-A-03/078435
- WO-A-2008/078091
- FR-A- 2 925 900
- US-A1- 2008 207 902
- SCHWEIZER, EDWARD E. ET AL: "Reactions of azines. 9. Preparation of 4,5-dihydropyrazolo[1,5-a]pyridines, 6,7-dihydropyrazolo[1,5-a]pyridines, and pyrazolo[1,5-a]pyridines", JOURNAL OF ORGANIC CHEMISTRY , 52(7), 1319-24 CODEN: JOCEAH; ISSN: 0022-3263, 1987, XP002566492,

## Description

La présente invention se rapporte à des dérivés de 5-phényl-pyrazolopyridine, à leur préparation et à leur application en thérapeutique dans le traitement ou la prévention de maladies impliquant les récepteurs nucléaires Nurr-1, aussi appelés NR4A2, NOT, TINUR, RNR-1, et HZF3.

Il est connu du document WO 2008/078091 la préparation ainsi que la mise en oeuvre de dérivés de phényle-imidazopyridine et de phényle-pyrazolopyrimidine à titre d'inhibiteurs du récepteur au FGF.

La présente invention a pour objet les composés de formule (I) : 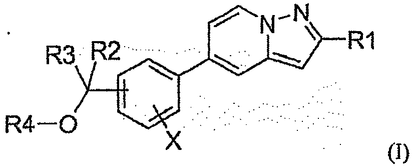 dans laquelle :
R₁ représente :
   un groupe phényle ou un groupe naphthyle, ces deux groupes pouvant éventuellement être substitués par un ou plusieurs atomes ou groupes choisis indépendamment les uns des autres parmi les atomes ou groupes suivants : halogène, (C₁-C₆)alkyle, halo(C₁-C₆)alkyle, (C₁-C₆)alcoxy, halo(C₁-C₆)alcoxy, (C₁-C₆)thioalkyle, -S(O)(C₁-C₆)alkyle, -S(O)₂(C₁-C₆-alkyle), hydroxyle, hydroxy(C₁-C₆)alkylène, CHO, COOH, (C₁-C₆)alcoxy(C₁-C₆)alkylène-oxy, NRaRb, CONRaRb, SO₂NRaRb, NRcCORd, OC(O)NRaRb, OCO(C₁-C₆)alkyle, NRcC(O)ORe, NRcSO₂Re,
X représente de 1 à 4 substituants identiques ou différents l'un de l'autre choisis parmi hydrogène, halogène, (C₁-C₆)alkyle, (C₁-C₆)alcoxy, le groupe (C₁-C₆)alkyle pouvant être éventuellement substitué par un ou plusieurs groupes choisis parmi un halogène, (C₁-C₆)alcoxy, hydroxyle,
R₂ et R₃ représentent, indépendamment l'un de l'autre,
   un atome d'hydrogène,
   un groupe (C₁-C₆)alkyle éventuellement substitué par un groupe Rf ;
   un groupe CHO ou COOH,
X et R3 peuvent former ensemble avec les atomes de carbone qui les portent un cycle carboné de 5 à 7 atomes de carbone ;
R₄ représente un atome d'hydrogène ou un groupe (C₁-C₆)alkyle,
Ra et Rb représentent, indépendamment l'un de l'autre, un atome d'hydrogène ou un groupe (C₁-C₆)alkyle, aryl(C₁-C₆)alkylène ou aryle ;
ou Ra et Rb forment ensemble, avec l'atome d'azote qui les porte, un groupe azétidine, pyrrolidine, pipéridine, azépine, morpholine, thiomorpholine, pipérazine, homopipérazine,
ce groupe étant éventuellement substitué par un groupe (C₁-C₆)alkyle, aryle ou aryl(C₁-C₆)alkylène ;
Rc et Rd représentent, indépendamment l'un de l'autre, un atome d'hydrogène ou un groupe (C₁-C₆)alkyle, aryl(C₁-C₆)alkylène, aryle ;
ou Rc et Rd forment ensemble un groupe (C₂-C₅)alkylène ;
Re représente un groupe (C₁-C₆)alkyle, aryl(C₁-C₆)alkylène, aryle ;
ou Rc et Re forment ensemble un groupe (C₂-C₅)alkylène ;
Rf représente un groupe-hydroxyle, un groupe oxo, CHO ou COOH,
à l'état de base ou de sel d'addition à un acide.

Les composés de formule (I) peuvent comporter un ou plusieurs atomes de carbone asymétriques. Ils peuvent donc exister sous forme d'énantiomères ou de diastéréoisomères. Ces énantiomères, diastéréoisomères, ainsi que leurs mélanges, y compris les mélanges racémiques, font partie de l'invention.

Les composés de formule (I) peuvent exister à l'état de bases ou de sels d'addition à des acides. De tels sels d'addition font partie de l'invention.
Ces sels peuvent être préparés avec des acides pharmaceutiquement acceptables, mais les sels d'autres acides utiles, par exemple, pour la purification ou l'isolement des composés de formule (I) font également partie de l'invention.
Les composés de formule (I) peuvent également exister sous forme d'hydrates ou de solvats, à savoir sous forme d'associations ou de combinaisons avec une ou plusieurs molécules d'eau ou avec un solvant. De tels hydrates et solvats font également partie de l'invention.
Dans le cadre de la présente invention, on entend par :
- un groupe (Cₓ-Cₜ) : un groupe comprenant entre x et t atomes de carbone ;
- un atome d'halogène : un fluor, un chlore, un brome ou un iode ;
- un groupe alkyle : un groupe aliphatique saturé linéaire, ramifié ou cyclique, éventuellement substitué par un groupe alkyle saturé linéaire, ramifié ou cyclique. A titre d'exemples, on peut citer les groupes méthyle, éthyle, propyle, isopropyle, butyle, isobutyle, tertbutyle, cyclopropyle, cyclobutyle, cyclopentyle, cyclohexyle, méthylcyclopropyle, cyclopropylméthyle etc ;
- un groupe alkylène : un groupe alkyle divalent ;
- un groupe alcoxy : un radical -O-alkyle où le groupe alkyle est tel que précédemment défini ;
- un groupe haloalkyle : un groupe alkyle substitué par un ou plusieurs atomes d'halogène identiques ou différents. A titre d'exemples, on peut citer les groupes CF₃, CH₂CF₃, CHF₂, CCl₃.
- un groupe haloalcoxy : un radical -O-alkyle où le groupe alkyle est tel que précédemment défini et substitué par un ou plusieurs atomes d'halogène identiques ou différents. A titre d'exemples, on peut citer les groupes OCF₃, OCHF₂, OCCl₃.
- un groupe thioalkyle : un radical S-alkyle où le groupe alkyle est tel que précédemment défini ;
- les atomes de soufre et d'azote peuvent être à l'état oxydé (N-oxide, sulfoxide, sulfone) ;
- un groupe aryle : groupe mono ou bicyclique aromatique comportant de 6 à 10 atomes. A titre d'exemples de groupes aryles, on peut citer : phényle et naphthyle.
- un cycle carboné : groupe mono ou bicyclique saturé, partiellement saturé ou insaturé comportant de 5 à 7 atomes de carbone. A titre d'exemples de cycles carbonés, on peut citer : indane.

Parmi les composés de formule (I) objets de l'invention, un premier groupe de composés est constitué des composés pour lesquels :
R1 représente un groupe phényle substitué par un halogène ;
R2 et R3 représentent indépendamment l'un de l'autre un atome d'hydrogène, un groupe (C₁-C₆)alkyle,
R4 représente un atome d'hydrogène ou un groupe (C₁-C₆)alkyle;
X représente un ou plusieurs atomes d'hydrogène ou d'halogène, ou X et R3 peuvent former ensemble avec les atomes de carbone qui les portent un cycle carboné de 5 atomes de carbone ;
à l'état de base ou de sel d'addition à un acide.

Parmi les composés de formule (I) objets de l'invention, un second groupe de composés est constitué des composés pour lesquels :
R1 représente un groupe phényle substitué par un atome de chlore ou de fluor ;
R2 et R3 représentent indépendamment l'un de l'autre un atome d'hydrogène, un méthyle, ou un groupe cyclopropyle ;
R4 représente un atome d'hydrogène ou un groupe méthyle;
X représente un ou plusieurs atomes d'hydrogène ou de fluor, ou X et R3 peuvent former ensemble avec les atomes de carbone qui les portent et avec le cycle benzofusionné un groupe indane,
à l'état de base ou de sel d'addition à un acide.

Parmi les composés de formule (I) objets de l'invention, on peut notamment citer les composés suivants :
{3-[2-(4-Chlorophényl)pyrazolo[1,5-*α*]pyridin-5-yl]phényl}méthanol
2-{3-[2-(4-Chlorophényl)pyrazolo[1,5-*α*]pyridin-5-yl]phényl}propan-2-ol
1-{3-[2-(4-Chlorophényl)pyrazolo[1,5-*α*]pyridin-5-yl]phényl}éthanol
1-{3-[2-(4-Chlorophényl)pyrazolo[1,5-*α*]pyridin-5-yl]phényl}cyclopropyl Méthanol
{3-[2-(4-Chlorophényl)pyrazolo[1,5-*α*]pyridin-5-yl]-2,6-difluorophényl} méthanol
{3-[2-(4-Fluorophényl)pyrazolo[1,5-*α*]pyridin-5-yl]phényl}méthanol
2-{3-[2-(4-Fluorophényl)pyrazolo[1,5-*α*]pyridin-5-yl]phényl}propan-2-ol
1-{3-[2-(4-Fluorophényl)pyrazolo[1,5-*α*]pyridin-5-yl]phényl}éthanol
2-((4-Chlorophényl)-5-(3-méthoxyméthylphényl)pyrazolo[1,5-*α*]pyridine
{4-[2-(4-Chlorophényl)pyrazolo[1,5-*α*]pyridin-5-yl]phényl}méthanol
{2-[2-(4-Chlorophényl)pyrazolo[1,5-*α*]pyridin-5-yl]phényl} méthanol
6-[2-(4-Chlorophényl)pyrazolo[1,5-*α*]pyridin-5-yl]indan-1-ol

Conformément à l'invention, on peut préparer les composés de formule générale (I) selon le procédé décrit dans le schéma 1.

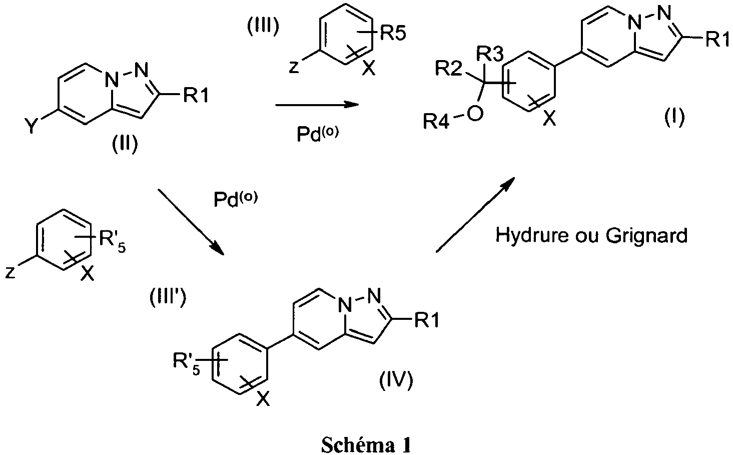

On peut préparer les composés de l'invention suivant le schéma 1 par une réaction de couplage, catalysée par un métal tel que le palladium, entre une pyrazolopyridine de formule générale (II), dans laquelle R1 est défini comme précédemment et Y représente un atome d'halogène ou un dérivé de bore, et un dérivé de formule générale (III) dans laquelle X est défini comme précédemment, Z représente un dérivé de bore ou d'étain si Y représente un atome d'halogène, ou bien un atome d'halogène si Y représente un dérivé de bore, et R5 représente le groupe 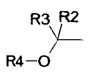 pour obtenir les composés de formule générale (I), par exemple, selon la méthode décrite par A. Gueiffier dans Helv. Chim. Acta, 2001, 84, 3610-3615.

On peut aussi préparer les composés de l'invention suivant le schéma 1 par une réaction de couplage, catalysée par un métal tel que le palladium, entre une pyrazolopyridine de formule générale (II), dans laquelle R1 est défini comme précédemment et Y représente un atome d'halogène ou un dérivé de bore, et un dérivé de formule générale (III') dans laquelle X est défini comme précédemment, Z représente un dérivé de bore ou d'étain si Y représente un atome d'halogène, ou bien un atome d'halogène si Y représente un dérivé de bore, et R5' représente un dérivé carbonylé R₂CO, dans lequel R2 est défini comme précédemment ou bien R5' représente un carboxylate d'alkyle ou un aldéhyde, pour obtenir les composés de formule générale (N), par exemple, selon la méthode décrite par A. Gueiffier dans Helv. Chim. Acta 2001, 84, 3610-3615.

Ensuite, on peut transformer les composés de formule générale (IV) en composés de formule générale (I), pour lesquels R4 représente un atome d'hydrogène, par action d'un dérivé organométallique tel qu'un organomagnésien, par exemple R3MgBr dans lequel R3 est défini comme précédemment, ou par réduction du groupement carbonyle au moyen d'un hydrure métallique, par exemple le borohydrure de sodium ou un de ses dérivés, ou toute autre méthode connue de l'Homme du métier.

Conformément à l'invention, on peut préparer les composés de formule générale (IIa), selon le procédé décrit dans le schéma 2.

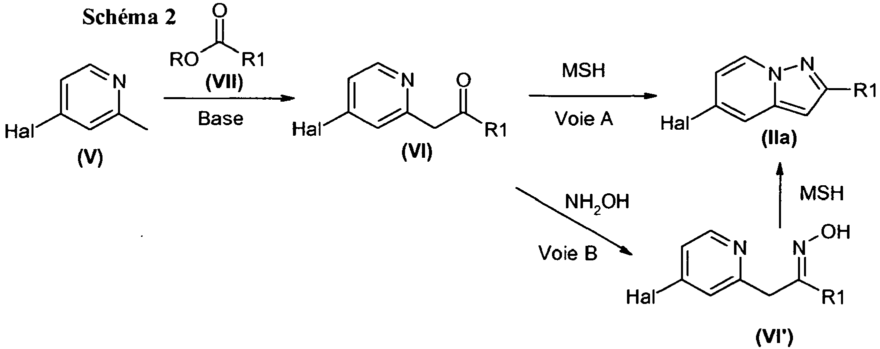

Dans le schéma 2 voie A, les composés de formule générale (IIa) dans laquelle R1 est défini comme précédemment et Hal représente un atome d'halogène peuvent être préparés par action du *O*-(mésitylènesulfonyl)hydroxylamine (MSH) sur un composé de formule générale (VI) dans laquelle R1 et Hal sont définis comme précédemment, par exemple selon la méthode décrite par Y. Tamura, J.-H. Kim, Y. Miki, H. Hayashi, M. Ikeda, dans J.Het. Chem., 1975, 12, 481.

Dans le schéma 2 voie B, on peut également préparer les composés de formule générale (IIa) dans laquelle R1 est défini comme précédemment et Hal représente un atome d'halogène par action du *O*-(mésitylènesulfonyl)hydroxylamine sur une oxime de formule générale (VI') dans laquelle R1 et Hal sont définis comme précédemment, par exemple selon la méthode décrite par Y. Tamura, J.-H. Kim, Y. Miki, H. Hayashi, M. Ikeda, dans J.Het. Chem., 1975, 12, 481. Les composés (VI') peuvent être obtenus par action de l'hydroxylamine sur les composés (VI).

Les composés (VI) peuvent être obtenus à partir des composés (V) par action des esters de formule générale (VII) dans laquelle R1 est défini comme précédemment et R représente un groupement alkyle, en présence d'une base forte, par exemple selon la méthode décrite par K.S. Gudmundsson dans Bioorg. Med. Chem., 2005, 13, 5346.

Conformément à l'invention, on peut également préparer les composés de formule générale (IIb), selon le procédé décrit dans le schéma 3.

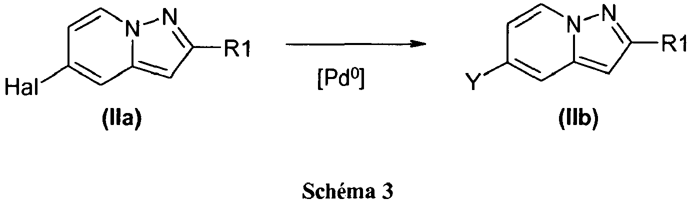

On peut préparer les composés (IIb) dans lesquels Y représente un dérivé de bore suivant le schéma 3 par une réaction de couplage, par exemple du bis(pinacolato)dibore, sur les composés (Ha), catalysée par un métal tel que le palladium selon la méthode décrite par E.F. DiMauro, R.Vitullo, J.Org.Chem., 2006, 71(10), 3959.

Dans les schémas 1, 2 et 3, les composés de départ et les réactifs, quand leur mode de préparation n'est pas décrit, sont disponibles dans le commerce ou décrits dans la littérature, ou bien peuvent être préparés selon des méthodes qui y sont décrites ou qui sont connues de l'Homme du métier.

L'invention, selon un autre de ses aspects, a également pour objet les composés de formule (IIb), notamment le composé de formule (IIb1). Ces composés sont utiles comme intermédiaires de synthèse des composés de formule (I).

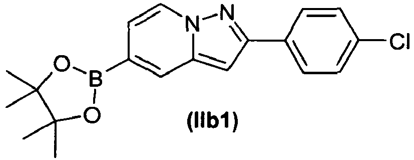

Les exemples suivants décrivent la préparation de certains composés conformes à l'invention. Ces exemples ne sont pas limitatifs et ne font qu'illustrer la présente invention. Les numéros des composés exemplifiés renvoient à ceux donnés dans le tableau ci-après, qui illustre les structures chimiques et les propriétés physiques de quelques composés selon l'invention.

### Exemple 1 : {3-[2-(4-Chlorophényl)pyrazolo[1,5-α]pyridin-5-yl]phényl}méthanol (composé 1 du tableau)

### 1.1 2-(4-Bromopyridin-2-yl)-1-(4-chlorophényl)éthanone

Sous courant d'argon, 1,0 g (5,8 mmol) de 4-bromo-2-méthylpyridine et 2,14 g (11,6 mmol) de 4-chlorobenzoate d'éthyle sont placés dans un ballon et dissous dans 10 mL de tétrahydrofurane anhydre. On refroidit à 0°C et on ajoute 12 mL d'une solution d'hexaméthyldisilazane de lithium (LiHMDS) (1M dans le tétrahydrofurane). Après addition, le mélange est chauffé à 45°C pendant 3h, refroidi à température ambiante, puis de l'eau est additionnée. Le tétrahydrofurane est ensuite évaporé sous pression réduite et la phase aqueuse est extraite trois fois à l'éther. La phase organique est séparée, séchée et concentrée sous pression réduite. Le résidu obtenu est purifié par chromatographie sur gel de silice en éluant avec un mélange de dichlorométhane et d'heptane. On obtient 1,11 g (62%) de composé.
LC-MS : M+H = 310
RMN-¹H (DMSO) δ (ppm) : 4,6 (s, 2H) ; 6,4 (s, 1H) ; 7,4 (s, 1H) ; de 7,5 à 7,6 (m, 6H) ; 7,7 (s, 1H) ; 7,9 (d, 2H); 8,1 (d,2H) ; 8,3 (d, 1H) ; 8,4 (d, 1H) ; 15,0 (s, 1H). (ratio *céto* / *énol:* 43 / 57).

### 1.2 O-Mésitylènesulfonylhydroxylamine

On place dans un ballon 5,00 g (7,52 mmol) de O-(2-mésitylènesulfonyl)acétohydroxamate d'éthyle dans 3 mL de 1,4-dioxane. On refroidit à 0°C et ajoute 2 mL (23,20 mmol) d'acide perchlorique HClO₄ (70% dans l'eau). Le mélange est ensuite agité pendant 15 minutes à 0°C et on y ajoute de l'eau glacée. Il se forme un précipité que l'on recueille par filtration, lave à l'eau glacée et à l'éther de pétrole froid. On obtient 5,26 g de composé.
RMN-¹H (DMSO) δ (ppm) : 2,2 (s, 3H) ; 2,5 (s, 6H) ; 6,8 (s, 2H) ; 9,0 (s, 2H). M+H = 216.

### 1.3 5-bromo-2-(4-chlorophényl)pyrazolo[1,5-α]pyridine

705 mg (3,27 mmol) d'*O*-mésitylènesulfonylhydroxylamine (composé obtenu selon le protocole décrit en 1.2) sont placés dans un ballon et dissous dans 10 mL de dichlorométhane. On refroidit à 0°C et on ajoute goutte à goutte une solution de 705 mg (2,27 mmol) de 2-(4-bromopyridin-2-yl)-1-(4-chlorophényl)éthanone (composé obtenu selon le protocole décrit en 1.1) dans 14 mL de dichlorométhane. Après 1 heure d'agitation à 0°C et 6 heures à température ambiante, on ajoute au mélange réactionnel, 150 mg d'*O-*(mésitylènesulfonyl)hydroxylamine dans 3 mL de dichlorométhane et on agite 16 h à température ambiante. Puis, on ajoute de nouveau 300 mg d'*O-*(mésitylènesulfonyl)hydroxylamine dans 1 mL de dichlorométhane au mélange et on laisse agiter à température ambiante pendant 7 heures. Il se forme un précipité qu'on élimine par filtration. Puis le filtrat est lavé successivement à l'eau, avec une solution aqueuse d'hydrogénocarbonate de sodium et par une solution saturée de chlorure de sodium, puis séché sur sulfate de magnésium et concentré sous pression réduite. Le résidu obtenu est purifié par chromatographie sur gel de silice en éluant avec du dichlorométhane. On obtient 126 mg (18%) de composé.
LC-MS : M+H = 307
RMN-¹H (DMSO) δ (ppm) : 7,0 (d, 1H) ; 7,1 (s, 1H) ; 7,6 (d, 2H) ; 8,0 (s, 1H) ; 8,1 (d, 2H) ; 8,7 (d, 1H).

### 1.4 {3-[2-(4-chlorophényl)pyrazolo[1,5-α]pyridin-5-yl]phényl}méthanol (composé n° 1)

Sous un courant d'argon, 126 mg (0,41 mmol) de 5-bromo-2-(4-chlorophényl)pyrazolo[1,5-*α*]pyridine (composé obtenu selon le protocole décrit en 1.3), 80 mg (0,53 mmol) d'acide 3-(hydroxyméthyl)phénylboronique et 24 mg (0,02 mmol) de tétrakis(triphénylphosphine)palladium sont placés dans un ballon contenant un mélange de 4 mL de diméthoxyéthane et 1 mL d'une solution aqueuse de carbonate de sodium (2M) préalablement dégazé sous courant d'argon. Le mélange réactionnel est chauffé à 80°C pendant 6 heures, puis, après refroidissement, concentré sous pression réduite. Le résidu est repris entre l'acétate d'éthyle et l'eau puis la phase organique est séparée, séchée et concentrée sous pression réduite. Le résidu est purifié par chromatographie sur gel de silice en éluant avec un mélange de dichlorométhane et de méthanol. Le solide obtenu est trituré à l'éther éthylique, recueilli par filtration puis séché à l'étuve sous pression réduite. On obtient 57 mg (41%) de composé.
Point de fusion (°C) : 161 - 164.
LC-MS : M+H = 335
RMN-¹H (DMSO) δ (ppm): 4,6 (d, 2H) ; 5,3 (t, 1H) ; 7,1 (s, 1H) ; 7,3 (d, 1H) ; 7,4 (d, 1H) ; 7,5 (t, 1H) ; 7,6 (d, 2H) ; 7,7 (d, 1H) ; 7,8 (s, 1H) ; 8,0 (s, 1H) ; 8,1 (d, 2H) ; 8,8 (d, 1H).

### Exemple 2 : 2-(4-chlorophényl)-5-(3-méthoxyméthylphényl)pyrazolo[1,5-α]pyridine (composé 9 du tableau)

### 2.1 2-(4-bromopyridin-2-yl)-1-(4-chlorophényl)éthanone

Sous courant d'azote, 5 g (29,07 mmol) de 4-bromo-2-méthylpyridine et 11,27 g (61,04 mmol) de 4-chlorobenzoate d'éthyle sont placés dans un ballon et dissous dans 50 mL de tétrahydrofurane anhydre. On refroidit à 5°C et on ajoute goutte à goutte 70 mL (70 mmol) d'une solution d'hexaméthyldisilazane de lithium (1M dans le tétrahydrofurane). Après addition, le mélange est agité à température ambiante pendant 2h, refroidi à 5°C, puis 100 mL d'eau sont additionnés progressivement. Le milieu est ensuite dilué avec 250 mL d'acétate d'éthyle et 100 mL d'eau. La phase organique est séparée, la phase aqueuse est extraite deux fois avec 100 mL d'acétate d'éthyle. Les phases organiques sont ensuite réunies, séchées sur sulfate de sodium et filtrées. On ajoute ensuite au filtrat 15 g de silice, concentre sous pression réduite. La poudre obtenue est utilisée comme dépôt solide pour une chromatographie sur gel de silice avec pour éluant un mélange de cyclohexane et d'acétate d'éthyle (9/1). 8,4 g (93%) de composé sont obtenus sous forme d'une poudre jaune.
LC-MS : M+H = 310
RMN-¹H (DMSO) δ (ppm) : 4,6 (s, 2H) ; 6,4 (s, 1H) ; 7,4 (s, 1H) ; de 7,5 à 7,6 (m, 6H) ; 7,7 (s, 1H) ; 7,9 (d, 2H) ; 8,1 (d,2H) ; 8,3 (d, 1H) ; 8,4 (d, 1H) ; 15,0 (s, 1H) (mélange *cétone* / *énol:* 40 / 60).

### 2.2 2-(4-Bromopyridin-2-yl)-1-(4-chlorophényl)éthanone oxime

On place dans un ballon 8,4 g (27,05 mmol) de 2-(4-bromopyridin-2-yl)-1-(4-chlorophényl)éthanone dans 150 mL d'éthanol. On ajoute 22 mL (272,56 mmol) de pyridine et 7,5 g (107,93 mmol) d'hydroxylamine monochlorhydrate Le mélange est ensuite agité pendant 5 heures à température ambiante puis le milieu réactionnel est concentré sous pression réduite jusqu'à obtention d'un solide pâteux jaune que l'on reprend avec 400mL d'acétate d'éthyle et 400 mL d'eau. La phase organique est séparée, la phase aqueuse est extraite trois fois avec 200 mL d'acétate d'éthyle. Les phases organiques sont ensuite réunies, séchées sur sulfate de sodium et filtrées. Le filtrat est concentré sous pression réduite : on obtient 8,1 g (91,9%) de composé sous forme d'une poudre bleue.
LC-MS : M+H = 325
RMN-¹H (DMSO) δ (ppm) : 4,3 (s, 2H) ; 7,45 (m, 2H) ; 7,50 (d, 1H) ; 7,55 (s, 1H) ; 7,75 (m, 2H) ; 8,35 (d, 1H) ; 11,65 (s, 1H).

### 2.3. 5-bromo-2-(4-chlorophényl)pyrazolo[1,5-α]pyridine

On place dans un ballon 12,9 g (45,21 mmol) de *O*-(2-mésitylènesulfonyl)acétohydroxamate d'éthyle dans 30 mL de 1,4-dioxane. On refroidit à 0°C et on ajoute 13,5 mL (156,60 mmol) d'acide perchlorique (70% dans l'eau). On ajoute ensuite 10 mL de 1,4-dioxane puis le milieu est agité vigoureusement pendant 2h30 minutes à 0°C. Le milieu est ensuite versé dans 350 mL d'eau glacée. On laisse le milieu vers 0°C pendant 10 minutes puis on récupère par filtration sur verre fritté le solide blanc formé (ne pas sécher totalement, le produit est potentiellement explosif à l'état sec). Le solide pâteux blanc obtenu est lavé avec 350 mL d'eau glacée puis est repris avec 250 mL de 1,2-dichloroéthane et 150mL de saumure refroidie vers 5°C. On récupère la phase organique que l'on filtre sur cartouche hydrophobe (Colonne d'extraction liquide/liquide 70 mL, Radleys®). On récupère le filtrat que l'on ajoute goutte à goutte sur une solution refroidie vers 0°C de 8,1g (24,88 mmol) de 2-(4-bromopyridin-2-yl)-1-(4-chlorophényl)éthanone oxime (composé obtenu dans l'étape 2.2) dans 150mL de 1,2-dichloroéthane.

Après l'ajout, on laisse revenir et on agite à température ambiante pendant 3 heures. On ajoute ensuite successivement au milieu 250 mL de dichlorométhane, 200 mL d'eau et 100 mL d'une solution aqueuse NaOH (1N). On laisse agiter puis on décante. La phase organique est séparée et la phase aqueuse est extraite avec 2 fois 200 mL de dichlorométhane. Les phases organiques sont ensuite réunies, filtrées sur cartouche hydrophobe (Colonne d'extraction liquide/liquide 70 mL, Radleys®) puis mélangées avec 15 g de silice. Le filtrat est ensuite concentré sous pression réduite : on obtient une poudre marron que l'on utilise comme dépôt solide pour une chromatographie sur gel de silice en éluant avec un mélange de cyclohexane et de dichlorométhane (1/1). On obtient 5,8 g (75%) de composé sous forme d'un solide cotonneux légèrement jaune.
LC-MS : M+H = 307
RMN-¹H (DMSO) δ (ppm) : 7,0 (d, 1H) ; 7,1 (s, 1H) ; 7,6 (d, 2H) ; 8,0 (s, 1H) ; 8,1 (d, 2H) ; 8,7 (d, 1H).

### 2.4. 2-(4-chlorophényl)-5-(3-méthoxyméthylphényl)pyrazolo[1,5-α]pyridine (composé n° 9)

150m g (0,49 mmol) de 5-bromo-2-(4-chlorophényl)pyrazolo[1,5-*α*]pyridine, obtenu selon le protocole décrit dans les exemples 1.3 ou 2.3, 0,900 g (0,54 mmol) d'acide 3-méthoxyméthylphénylboronique , 0,475 g (1,46 mmol) de carbonate de césium et 0,04 g (0,05 mmol) de [1,1'-bis(diphénylphosphino)ferrocène]dichloropalladium (II) sont placés dans un réacteur contenant 5 mL d'un mélange THF-eau (9/1). Le milieu est porté à 70°C pendant 2 heures puis est remis à température ambiante. Le milieu est ensuite dilué avec 40 mL de dichlorométhane et 40 mL d'eau. Le milieu est filtré sur cartouche hydrophobe (Colonne d'extraction liquide/liquide 70 mL, Radleys®) et on ajoute 2g de silice au filtrat obtenu. Après concentration sous pression réduite, le résidu obtenu est purifié par chromatographie sur gel de silice en éluant avec un mélange de cyclohexane et d'acétate d'éthyle (7/3). On obtient 0,14 g (82%) de produit attendu sous la forme d'une poudre jaune pâle.
Point de fusion (°C) : 130-132.
LC-MS : M+H = 349
RMN-¹H (DMSO) δ (ppm) : 3,35 (s, 3H) ; 4,55 (s, 2H) ; 7,15 (s, 1H) ; 7,30 (d, 1H) ; 7,40 (s, 1H) ; 7,55 (m, 3H) ; 7,80 (m, 2H) ; 8,05 (m, 3H) ; 8,80 (d, 1H).

### Exemple 3 : {4-[2-(4-chlorophényl)pyrazolo[1,5-α]pyridin-5-yl]phényl}méthanol (composé 10 du tableau)

300 mg (0,98 mmol) de 5-bromo-2-(4-chlorophényl)pyrazolo[1,5-*α*]pyridine (composé obtenu selon le protocole décrit dans les exemples 1.3 ou 2.3, 180 mg (1,18 mmol) d'acide 4-(hydroxyméthyl)phénylboronique, 975 mg (2,99 mmol) de carbonate de césium et 85 mg (0,10 mmol) de [1,1'-bis(diphénylphosphino)ferrocène]dichloropalladium (II) sont placés dans un tube réacteur contenant 5 mL d'un mélange THF-eau (9-1). Le mélange réactionnel est chauffé à 60°C pendant 2 heures, puis remis à température ambiante. Le milieu réactionnel est alors dilué avec 50 mL d'eau et 50 mL de dichlorométhane puis filtré sur cartouche hydrophobe (Colonne d'extraction liquide/liquide 70 mL, Radleys®). 2 g de silice sont ajoutés au filtrat avant de le concentrer sous pression réduite. La poudre marron obtenue est utilisée comme dépôt solide pour une chromatographie sur gel de silice en éluant avec un mélange de cyclohexane et d'acétate d'éthyle (7/3). On obtient 262 mg (80%) de composé sous forme d'une poudre grise.
Point de fusion (°C) : 214-216.
LC-MS : M+H = 335
RMN-¹H (DMSO) δ (ppm) : 4,60 (d, 2H) ; 5,30 (d, 1H) ; 7,20 (s, 1H) ; 7,30 (d, 1H) ; 7,45 (d, 2H) ; 7,60 (d, 2H) ; 7,80 (d, 2H) ; 8,05 (m, 3H) ; 8,80 (d, 1H).

### Exemple 4 : {2-[2-(4-chlorophényl)pyrazolo[1,5-α]pyridin-5-yl]phényl}méthanol (composé 11 du tableau)

On procède suivant le mode opératoire décrit dans l'exemple 3. A partir de 0,3 g (0,98 mmol) de 5-bromo-2-(4-chlorophényl)pyrazolo[1,5-*α*]pyridine, obtenu selon le protocole décrit dans les exemples 1.3 ou 2.3, de 0,18 mg (1,18 mmol) d'acide 2-(hydroxyméthyl)phénylboronique, de 0,975 g (2,99 mmol) de carbonate de césium, de 0,085 g (0,10 mmol) de [1,1'-bis(diphénylphosphino)ferrocène]dichloropalladium (II) et de 5 mL d'un mélange THF-eau (9/1) et après chromatographie sur gel de silice en éluant avec un mélange de cyclohexane et d'acétate d'éthyle (7/3), on obtient 0,303 g (92%) de produit attendu sous la forme d'une poudre blanche.
Point de fusion (°C) : 144-146.
LC-MS : M+H = 335
RMN-¹H (DMSO) δ (ppm) : 4,50 (d, 2H) ; 5,25 (d, 1H) ; 7,00 (d, 1H) ; 7,15 (s, 1H) ; de 7,40 à 7,50 (m, 3H) ; de 7,55 à 7,65 (m, 3H) ; 7,75 (s, 1H) ; 8,05 (d, 2H) ; 8,75 (d, 1H).

### Exemple 5 : {3-[2-(4-fluorophényl)pyrazolo[1,5-α]pyridin-5-yl]phényl}méthanol (composé 6 du tableau)

### 5.1 2-(4-bromopyridin-2-yl)-1-(4-fluorophényl)éthanone

Sous courant d'azote, 5,0 g (29,07 mmol) de 4-bromo-2-picoline et 10,2 g (60,95 mmol) de 4-fluorobenzoate d'éthyle sont placés dans un ballon et dissous dans 50 mL de tétrahydrofurane anhydre. On refroidit à 0°C et on ajoute goutte à goutte 70 mL (70 mmol) d'une solution d'hexaméthyldisilazane de lithium (1M dans le tétrahydrofurane). Après addition, le mélange est agité à température ambiante pendant 2h, refroidi à 5°C, puis 100 mL d'eau sont additionnés progressivement. Le milieu est ensuite dilué avec 250 mL d'acétate d'éthyle et 100 mL d'eau. La phase organique est séparée, la phase aqueuse est extraite deux fois avec 100 mL d'acétate d'éthyle. Les phases organiques sont ensuite réunies, séchées sur sulfate de sodium et filtrées. On ajoute ensuite au filtrat 15 g de silice, agite puis concentre sous pression réduite. La poudre obtenue est utilisée comme dépôt solide pour une chromatographie sur gel de silice avec pour éluant un mélange de cyclohexane et d'acétate d'éthyle (9/1). 7,5 g (88%) de composé sont obtenus sous forme d'une poudre jaune.
LC-MS : M+H = 294 (ratio *cétone* / *énol:* 43 / 57)
RMN-¹H (DMSO) δ (ppm) : 4,56 (s, 2H) ; 6,34 (s, 1H) ; de 7,23 à 7,40 (m, 5H) ; 7,53 (d, 1H) ; 7,56 (m, 1H) ; 7,70 (d, 1H) ; de 7,81 à 7,92 (m, 2H) ; de 8,04 à 8,16 (m,2H) ; 8,29 (d, 1H) ; 8,37 (d, 1H) ; 15,0 (s, 1H).

### 5.2 2-(4-bromopyridin-2-yl)-1-(4-fluorophényl)éthanone oxime

7,5 g (24,26 mmol) de 2-(4-bromopyridin-2-yl)-1-(4-fluorophényl)éthanone sont placés dans un ballon contenant 100 mL d'éthanol absolu. 20 mL (247,78 mmol) de pyridine et 7,08 g (101,88 mmol) d'hydroxylamine monochlorhydrate sont ajoutés avant de laisser agiter le milieu pendant 3h à température ambiante. L'éthanol est ensuite évaporé sous vide et le résidu obtenu est repris avec 250 mL d'eau et 250 mL d'acétate d'éthyle. La phase organique est séparée, puis on extrait la phase aqueuse 5 fois avec 150 mL d'acétate d'éthyle. Les phases organiques sont ensuite réunies, séchées sur sulfate de sodium et concentrée sous vide. 7,82 g de composé sont obtenus.
LC-MS : M+H = 309
RMN 1H (DMSO-d6, δ en ppm): 4,26 (s, 2H) ; 7,19 (t, 2H) ; 7,50 (m, 2H) ; 7,75 (m, 2H) ; 8,33 (d, 1H) ; 11,50 (s, 1H). (obtention de l'oxime (E)).

### 5.3 5-bromo-2-(4-fluororophényl)pyrazolo[1,5-α]pyridine

7,82 g (25,50 mmol) de 2-(4-bromopyridin-2-yl)-1-(4-fluorophényl)éthanone oxime sont placés dans un ballon et dissous dans 400 mL de 1,2-dichloroéthane. Une solution de *O-*(mésitylènesulfonyl)hydroxylamine (0,27 M dans le 1,2-dichloroéthane - composé obtenu selon le protocole décrit en 1.3) est ajoutée goutte à goutte au milieu refroidi vers 0°C. Après l'ajout, le milieu est agité à température ambiante pendant 1h30. Le milieu est ensuite dilué avec 200mL d'eau et 200mL d'une solution de soude (1N). Le milieu biphasique est agité puis décanté. La phase organique est séparée, puis la phase aqueuse est extraite 4 fois avec 200 mL de dichlorométhane. Les phases organiques sont ensuite réunies, séchées sur sulfate de sodium et filtrées. On ajoute ensuite au filtrat 15 g de silice puis concentre sous pression réduite. La poudre obtenue est utilisée comme dépôt solide pour une chromatographie sur gel de silice avec pour éluant un mélange de cyclohexane et de dichlorométhane (1/1). 5,06 g (68%) de composé sont obtenus sous forme d'une poudre cotonneuse blanche.
LC-MS: M+H = 291
RMN 1H (DMSO-d6, δ en ppm): de 7,00 à 7,10 (m, 2H) ; 7,45 (m, 2H) ; 8,05 (m, 3H) ; 8,70 (d, 1H).

### 5.4 {3-[2-(4-fluorophényl)pyrazolo[1,5-α]pyridin-5-yl]phényl}méthanol (composé n°6)

On procède suivant le mode opératoire décrit dans l'exemple 3 à partir de 0,300 g (1,03 mmol) de 5-bromo-2-(4-fluorophényl)pyrazolo[1,5-*α*]pyridine, obtenu à l'étape 5.3, de 0,190 g (1,25 mmol) d'acide 3-(hydroxyméthyl)phénylboronique, de 1,00 g (3,07 mmol) de carbonate de césium, de 0,085 g (0,10 mmol) de [1,1'-bis(diphénylphosphino)ferrocène]dichloropalladium (II) et de 5 mL d'un mélange THF-eau (9/1) et après chromatographie sur gel de silice en éluant avec un mélange de cyclohexane et d'acétate d'éthyle (7/3), on obtient 0,245 g (74%) de produit attendu sous la forme d'une poudre blanche.
Point de fusion (°C) : 141-143.
LC-MS : M+H= 319
RMN-¹H (DMSO) δ (ppm) : 4,65 (d, 2H) ; 5,30 (t, 1H) ; 7,10 (s, 1H) ; de 7,25 à 7,40 (m, 4H) ; 7,50 (m, 1H) ; 7,70 (d, 1H) ; 7,80 (s, 1H) ; 8,00 (s, 1H) ; 8,05 (t, 2H) ; 8,80 (d, 1H).

### Exemple 6 : 1-{3-[2-(4-chlorophényl)pyrazolo[1,5-α]pyridin-5-yl]phényl}éthanol (composé 3 du tableau)

### 6.1 3-[2-(4-chlorophényl)pyrazolo[1,5-α]pyridin-5-yl]benzaldéhyde

Sous courant d'azote, sont introduits 0,300 g (0,98 mmol) de 5-bromo-2-(4-chlororophényl)pyrazolo[1,5-*α*]pyridine obtenu selon le protocole décrit dans les exemples 1.3 ou 2.3, 0,292 g (1,95 mmol) d'acide 3-formylphénylboronique, 0,94 g (2,88 mmol) de carbonate de césium dans 5 mL d'un mélange 9/1 de tétrahydrofurane et d'eau. 0,08 g (0,10 mmol) de [1,1'-bis(diphénylphosphino)ferrocène]dichloropalladium (II) sont ajoutés et le milieu est chauffé à 70°C pendant 2 heures. Le milieu est ensuite remis à température ambiante puis dilué avec du dichlorométhane et de l'eau. On filtre le milieu sur cartouche hydrophobe (Colonne d'extraction liquide/liquide 70 mL, Radleys®), récupère la phase organique à laquelle on ajoute 2 g de silice. Après évaporation du solvant, on purifie le résidu par chromatographie sur gel de silice en éluant avec un mélange de cyclohexane et d'acétate d'éthyle (9/1). On obtient 0,302 g (93%) de produit attendu sous la forme d'une poudre jaune.
Point de fusion (°C) : 152-154
LC-MS : M+H = 333
RMN-¹H (DMSO) δ (ppm) : 7,2 (s, 1H) ; 7,35 (d, 1H) ; 7,55 (d, 2H) ; 7,75 (t, 1H) ; 7,95 (d, 1H) ; 8,05 (d, 2H) ; 8,15 (s, 1H) ; 8,20 (d, 1H) ; 8,40 (s, 1H) ; 8,85 (d, 1H) ; 10,15 (s,1H). **6.2 1-{3-[2-(4-chlorophényl)pyrazolo[1,5-*α*]pyridin-5-yl]phényl}éthanol (composé 3 du tableau)**
Sous courant d'azote, 0,085 g (0,26 mmol) de 3-[2-(4-chlorophényl)pyrazolo[1,5-*α*]pyridin-5-yl]benzaldéhyde sont mis en solution dans 8 mL de tétrahydrofurane. Le milieu est refroidi à 5°C avant addition lente d'une solution de 0,30 mL (0,90 mmol) de bromure de méthylmagnésium (3 M dans l'éther éthylique). Le milieu est ensuite agité à température ambiante pendant 2 heures. On neutralise en ajoutant à froid et au goutte à goutte une solution aqueuse saturée en chlorure d'ammonium, puis dilué avec de l'acétate d'éthyle et de l'eau. La phase organique est séparée puis la phase aqueuse est extraite deux fois à l'acétate d'éthyle. Les phases organiques sont réunies, séchées sur sulfate de sodium, filtrées et concentrées sous vide. Le résidu est purifié par chromatographie sur gel de silice (par dépôt solide) en éluant avec un mélange de cyclohexane et d'acétate d'éthyle (8/2). On obtient 0,048 g (53%) de produit attendu sous la forme d'une poudre blanche.
Point de fusion (°C) : 93-95.
LC-MS: M+H = 349
RMN-¹H (DMSO) δ (ppm) : 1,45 (d, 3H) ; 4,85 (m, 1H) ; 5,25 (d, 1H) ; 7,15 (s, 1H) ; 7,30 (d, 1H) ; 7,45 (m, 2H) ; 7,60 (m, 2H) ; 7,70 (d, 1H) ; 7,80 (s, 1H) ; 8,05 (m, 3H) ; 8,80 (d, 1H).

### Exemple 7 : 1-{3-[2-(4-chlorophényl)pyrazolo[1,5-α]pyridin-5 yl] phényl}cyclopropyl méthanol (composé 4 du tableau)

On procède suivant le mode opératoire décrit dans l'étape 6.2. A partir de 0,15 g (0,45 mmol) de 3-[2-(4-chlorophényl)pyrazolo[1,5-*α*]pyridin-5-yl]benzaldéhyde obtenu selon le procédé décrit en 6.1, en solution dans 8 mL de tétrahydrofurane, puis addition d'une solution de 1,80 mL (0,9 mmol) de bromure de cyclopropylmagnésium (0,5M dans le tetrahydrofurane) et après chromatographie sur gel de silice en éluant avec un mélange de cyclohexane et d'acétate d'éthyle (8/2), on obtient 0,1 g (59 %) de produit attendu sous la forme d'une poudre beige.
Point de fusion (°C) : 141-143.
LC-MS : M+H = 375
RMN-¹H (DMSO) δ (ppm) : 0,45 (m, 4H) ; 1,15 (m, 1H) ; 4,10 (m, 1H) ; 5,25 (s, 1H) ; 7,15 (s, 1H) ; 7,30 (d, 1H) ; 7,50 (m, 2H) ; 7,60 (d, 2H) ; 7,70 (m, 1H) ; 7,80 (s, 1H) ; 8,00 (s, 1H) ; 8,10 (d, 2H) ; 8,80 (d, 1H).

### Exemple 8 : 1-{3-[2-(4-fluorophényl)pyrazolo[1,5-α]pyridin-5-yl]phényl}éthanol (composé 8 du tableau)

### 8.1 3-[2-(4-fluorophényl)pyrazolo[1,5-α]pyridin-5-yl]benzaldéhyde

Sous courant d'azote sont introduits 0,200 g (0,69 mmol) de 5-bromo-2-(4-fluorophényl)pyrazolo[1,5-*α*]pyridine obtenu à l'étape 5.3, 0,125 g (0,83 mmol) d'acide 3-formylphénylboronique, 0,670 g (2,06 mmol) de carbonate de césium dans 5 ml d'un mélange 9/1 de tétrahydrofurane et d'eau. 0,055 g (0,07 mmol) de [1,1'-bis(diphénylphosphino)ferrocène]dichloropalladium (II) sont ajoutés et le milieu est chauffé à 70°C pendant 3 heures. On rajoute ensuite 0,063 g (0,42 mmol) d'acide 3-formylphénylboronique, 0,335 g (1,03 mmol) de carbonate de césium et 0,028 g (0,03 mmol) de [1,1'-bis(diphénylphosphino)ferrocène]dichloropalladium (II) en laissant agiter à 70°C pendant 4h. Le milieu est ensuite remis à température ambiante puis dilué avec du dichlorométhane et de l'eau. On filtre le milieu biphasique sur cartouche hydrophobe (Colonne d'extraction liquide/liquide 70 mL, Radleys®), récupère la phase organique à laquelle on ajoute 1,2 g de silice. Après évaporation du solvant, on purifie le résidu par chromatographie sur gel de silice en éluant avec un mélange de cyclohexane et d'acétate d'éthyle (8/2). On obtient 0,175g (80%) de produit attendu sous la forme d'une poudre blanche.
LC-MS : M+H = 317
RMN-¹H (DMSO) δ (ppm) : 7,15 (s, 1H) ; de 7,30 à 7,37 (m, 3H) ; 7,77 (t,1H) ; 7,98 (m, 1H) ; 8,08 (m, 2H) ; 8,15 (m, 1H) ; 8,20 (m, 1H) ; 8,38 (s, 1H) ; 8,82 (d, 1H) ; 10,15 (s,1H).

### 8.2 1-{3-[2-(4-fluorophényl)pyrazolo[1,5-α]pyridin-5-yl]phényl}éthanol (composé 8 du tableau)

Sous courant d'azote 0,072 g (0,23 mmol) de 3-[2-(4-fluorophényl)pyrazolo[1,5-*α*]pyridin-5-yl]benzaldéhyde sont mis en solution dans 10 mL de tétrahydrofurane. Le milieu est refroidi à 0°C pour addition lente d'une solution de 0,23 mL (0,68 mmol) de bromure de méthylmagnésium (3M dans l'éther éthylique). Le milieu est ensuite remis à température ambiante et agité 1 heure 30 minutes. On neutralise en ajoutant à froid et au goutte à goutte une solution aqueuse saturée en chlorure d'ammonium, puis on dilue avec du dichlorométhane et de l'eau. On ajuste le pH du milieu à 9-10 par addition d'une solution aqueuse saturée en carbonate de sodium. Le milieu est filtré sur cartouche hydrophobe (Colonne d'extraction liquide/liquide 70 mL, Radleys®), la phase organique est récupérée et concentrée sous pression réduite après avoir ajouté 0,8 g de silice. Le résidu obtenu est purifié par chromatographie sur gel de silice en éluant le dépôt solide avec un mélange de cyclohexane et d'acétate d'éthyle (8/2). On obtient 0,056g (74%) de produit attendu sous la forme d'une poudre blanche.
Point de fusion (°C) : 132-134.
LC-MS : M+H = 333
RMN 1H (DMSO-d6, δ en ppm): 1,42 (d, 3H) ; 4.85 (q, 1H) ; 5,25 (s, 1H) ; 7,11 (s, 1H) ; 7,25 (d, 1H) ; 7,34 (m, 2H) ; de 7,40 à 7,50 (m, 2H) ; 7,69 (d, 1H) ; 7,80 (d, 1H) ; 8,00 (s, 1H) ; 8,08 (m, 2H) ; 8,75 (d, 1H).

### Exemple 9 : 2-{3-[2-(4-chlorophényl)pyrazolo[1,5-a]pyridin-5-yl]phényl}propan-2-ol (composé 2 du tableau)

### 9.1 3-[2-(4-chlororophényl)pyrazolo[1,5-α]pyridin-5-yl]benzoate de méthyle

On procède suivant le mode opératoire décrit dans l'exemple 3. A partir de 1,5 g (4,88 mmol) de 5-bromo-2-(4-chlorophényl)pyrazolo[1,5-*α*]pyridine, obtenu selon le protocole décrit dans les exemples 1.3 ou 2.3, 1,05 mg (5,83 mmol) d'acide 3-méthoxycarbonylphénylboronique , de 4,6 g (14,61 mmol) de carbonate de césium, de 0,400 g (0,49 mmol) de [1,1'-bis(diphenylphosphino)ferrocène]dichloropalladium (II) et de 20 mL d'un mélange THF-eau (9/1) et après chromatographie sur gel de silice en éluant avec un mélange de cyclohexane et d'acétate d'éthyle (9/1), on obtient 1,6 g (90 %) de produit attendu sous la forme d'une poudre jaune blanche.
Point de fusion (°C) : 180-182
LC-MS : M+H = 363
RMN-¹H (DMSO) δ (ppm) : 3,95 (s, 3H) ; 7,20 (s, 1H) ; 7,35 (d, 1H) ; 7,60 (d, 2H) ; 7,70 (t, 1H) ; de 8,00 à 8,20 (m, 5H) ; 8,35 (s, 1H) ; 8,85 (d, 1H).

### 9.2 2-{3-[2-(4-chlorophényl)pyrazolo[1,5-α]pyridin-5-yl]phényl}propan-2-ol (composé n°2)

Sous courant d'azote 0,13 g (0,36 mmol) de 3-[2-(4-chlororophényl)pyrazolo[1,5-*α*]pyridin-5-yl]benzoate de methyle sont mis en solution dans 8 mL de tétrahydrofurane. Le milieu est refroidi à 0°C pour addition lente d'une solution de 0,6 mL (1,80 mmol) de bromure de méthylmagnésium (3M dans l'éther éthylique). Le milieu est ensuite remis à température ambiante et agité 2 heures. On neutralise en ajoutant à froid et au goutte à goutte une solution aqueuse saturée en chlorure d'ammonium, puis on dilue le milieu avec 50 mL de dichlorométhane et 50 mL d'eau. Le milieu est filtré sur cartouche hydrophobe (Colonne d'extraction liquide/liquide 70 mL, Radleys®), la phase organique est récupérée et concentrée sous pression réduite après avoir ajouté 0,4 g de silice. Le résidu est purifié par chromatographie sur gel de silice en éluant le dépôt solide avec un mélange de cyclohexane et d'acétate d'éthyle (8/2). On obtient 0,08 g (61 %) de produit attendu sous la forme d'une poudre blanche.
Point de fusion (°C) : 159-161.
LC-MS : M+H = 363
RMN-¹H (DMSO) δ (ppm) : 1,50 (s, 6H) ; 5,10 (s, 1H) ; 7,15 (s, 1H) ; 7,30 (d, 2H) ; 7,45 (t, 1H) ; 7,55 (m, 2H) ; 7,70 (m, 1H) ; 7,90 (s, 1H) ; 8,00 (m, 1H) ; 8,05 (m, 2H) ;8,80 (d, 1H).

### Exemple 10: 2-{3-[2-(4-Fluorophényl)-pyrazolo[1,5-a]pyridin-5-yl]phényl}propan-2-ol (composé 7 du tableau)

### 10.1 3-[2-(4-fluorophényl)pyrazolo[1,5-a]pyridin-5-yl]benzoate de méthyle

Sous courant d'azote sont introduits 0,400 g (1,37 mmol) de 5-bromo-2-(4-fluorophényl)pyrazolo[1,5-*a*]pyridine obtenu à l'étape 5.3., 0,300 g (1,67 mmol) d'acide 3-méthoxycarbonylphénylboronique, 1,330 g (4,08 mmol) de carbonate de césium dans 5 ml d'un mélange 9/1 de tétrahydrofurane et d'eau. 0,11 g (0,13 mmol) de [1,1'-bis(diphenylphosphino)ferrocène]dichloropalladium (II) sont ajoutés et le milieu est chauffé à 70°C pendant 4 heures. Le milieu est ensuite remis à température ambiante puis dilué avec 40 mL de dichlorométhane et 40 mL d'eau. Le milieu est ensuite filtré sur cartouche hydrophobe (Colonne d'extraction liquide/liquide 70 mL, Radleys®), la phase organique est récupérée et concentrée sous pression réduite après avoir ajouté 2 g de silice. Le résidu par chromatographie sur gel de silice en éluant avec un mélange de cyclohexane et d'acétate d'éthyle (9/1). On obtient 0,340 g (71%) de produit attendu sous la forme d'une poudre blanche.
LC-MS : M+H = 347
RMN-¹H (DMSO) δ (ppm) : 3,95 (s, 3H) ; 7,15 (s, 1H) ; de 7,30 à 7,40 (m, 3H) ; 7,70 (t, 1H) ; de 8,00 à 8,15 (m, 5H) ; 8,35 (s, 1H) ; 8,80 (d, 1H).

### 10.2. 2-{3-[2-(4-fluorophényl)pyrazolo[1,5-a]pyridin-5-yl]phényl}propan-2-ol (composé n°7)

Sous courant d'azote 0,200 g (0,58 mmol) de 3-[2-(4-fluorophényl)pyrazolo[1,5-*a*]pyridin-5-yl]benzoate de méthyle sont mis en solution dans 15 mL de tétrahydrofurane. Le milieu est refroidi à 5°C pour addition lente d'une solution de 1 mL (3,00 mmol) de bromure de méthylmagnésium (3M dans l'éther éthylique). Le milieu est ensuite remis à température ambiante et agité 2 heures. On neutralise en ajoutant à froid et au goutte à goutte 10 mL d'une solution aqueuse saturée en chlorure d'ammonium, puis dilué avec 100 mL de dichlorométhane et 100 mL d'eau. La phase aqueuse est extraite avec deux fois 50 mL de dichlorométhane. Les phases organiques sont réunies, filtrées sur cartouche hydrophobe (Colonne d'extraction liquide/liquide 70 mL, Radleys®) et concentrées sous vide après avoir ajouté 2 g de silice. Le résidu est purifié par chromatographie sur gel de silice en éluant le dépôt solide avec un mélange de cyclohexane et d'acétate d'éthyle (7/3). On obtient 0,165 g (82%) de produit attendu sous la forme d'une poudre blanche.
Point de fusion (°C) : 162-164.
LC-MS : M+H = 347
RMN-¹H (DMSO) δ (ppm) : 1,50 (s, 6H) ; 5,10 (s, 1H) ; 7,10 (s, 1H) ; 7,25 (d, 1H) ; 7,35 (t, 2H) ; 7,45 (t, 1H) ; 7,55 (m, 1H) ; 7,65 (m, 1H) ; 7,90 (s, 1H) ; 8,00 (s, 1H) ; 8,10 (t, 2H) ; 8,80 (d, 1H).

### Exemple 11: {3-[2-(4-chlorophényl)pyrazolo[1,5-a]pyridin-5-yl]-2,6-difluorophényl} méthanol (composé 5 du tableau)

### 11.1. 3-[2-(4-chlorophényl)pyrazolo[1,5-a]pyridin-5-yl]-2,6-difluorobenzaldéhyde

Sous courant d'azote sont introduits 0,200 g (0,65 mmol) de 5-bromo-2-(4-chlorophényl)pyrazolo[1,5-*a*]pyridine obtenu selon le protocole décrit dans les exemples 1.3 ou 2.3, 0,145 g (0,78 mmol) d'acide 2,4-difluoro-3-formylphénylboronique, 0,640 g (1,96 mmol) de carbonate de césium dans 5 mL d'un mélange 9/1 de tétrahydrofurane et d'eau. 0,055 g (0,07 mmol) de [1,1'-bis(diphenylphosphino)ferrocène]dichloropalladium (II) sont ajoutés et le milieu est chauffé à 60°C pendant 2 heures. Le milieu est remis ensuite à température ambiante puis dilué avec 50 mL de dichlorométhane et 50 mL d'eau. On récupère la phase organique que l'on filtre sur cartouche hydrophobe (Colonne d'extraction liquide/liquide 70mL, Radleys®). 1,8 g de silice sont ajoutés au filtrat récupéré puis on concentre sous pression réduite. La poudre obtenue est purifiée par chromatographie sur gel de silice en éluant avec un mélange de cyclohexane et d'acétate d'éthyle (8/2). On obtient 0,195g (81%) de produit attendu sous la forme d'une poudre blanche.
Point de fusion (°C) : 187-189
LC-MS : M+H = 369
RMN-¹H (DMSO) δ (ppm) : 7,15 (d, 1H) ; 7,25 (s, 1H) ; 7,45 (t, 1H) ; 7,60 (d, 2H) ; 7,95 (s, 1H) ; 8,10 (m, 3H) ; 8,85 (d, 1H) ; 10,35 (s, 1H).

### 11.2. {3-[2-(4-chlorophényl)pyrazolo[1,5-a]pyridin-5-yl]-2,6-difluorophényl}méthanol (composé n°5)

A une solution de 0,135 g (0.37 mmol) de 3-[2-(4-chlorophényl)pyrazolo[1,5-*a*]pyridin-5-yl]-2,6-difluorobenzaldéhyde dans 10 mL d'un mélange de méthanol et de tetrahydrofurane (1-1) refroidie à environ 5°C, sont ajoutés par portions 0,040 g (1,06 mmol) de borohydrure de sodium. Le milieu est agité 1 heure à température ambiante puis refroidie à 5°C puis hydrolysé par addition goutte à goutte de 10 mL d'une solution aqueuse saturée en chlorure d'ammonium et 10 mL d'eau. On concentre sous pression réduite et le résidu obtenu est repris par 50 mL de dichlorométhane et 50 mL d'eau. Le milieu est ensuite filtré sur cartouche hydrophobe (Colonne d'extraction liquide/liquide 70 mL, Radleys®), la phase organique est récupérée et concentrée sous pression réduite après avoir ajouté 1 g de silice. On purifie le résidu par chromatographie sur gel de silice en éluant avec un mélange de cyclohexane et d'acétate d'éthyle (8/2). On obtient 0,065 g (48%) du produit attendu sous la forme d'une poudre blanche.
Point de fusion (°C) : 190-192.
LC-MS : M+H = 371
RMN-¹H (DMSO) δ (ppm) : 4,60 (d, 2H) ; 5,35 (t, 1H) ; 7,10 (d, 1H) ; 7,20 (s, 1H) ; 7,25 (t, 1H) ; 7,60 (d, 2H) ; 7,70 (m, 1H) ; 7,90 (s, 1H) ; 8,05 (d, 2H) ; 8,80 (d, 1H).

### Exemple 12: 6-[2-(4-chlorophényl)pyrazolo[1,5-a]pyridin-5-yl]indan-1-ol (composé 12 du tableau)

### 12.1. 2-(4-chlorophényl)-5-(4,4,5,5-tétraméthyl-[1,3,2]dioxaborolan-2-yl)pyrazolo[1,5-a]pyridine

On place dans 2 réacteurs micro-onde, 1,75g (5,69 mmol) de 5-bromo-2-(4-chlorophényl)pyrazolo[1,5-*a*]pyridine, 1,73g (6,82 mmol) de bis(pinacolato)dibore, 1,67g (17,03 mmol) d'acétate de potassium dans 15 mL de 1,4-dioxane. 930 mg (0,57 mmol) de [1,1'-bis(diphenylphosphino)ferrocène]dichloropalladium (II) sont alors ajoutés et les 2 milieux sont irradiés à 140°C pendant 20 minutes chacun.
Les 2 milieux réactionnels sont alors mélangés puis dilués avec 300mL de dichlorométhane et 200 mL d'eau. La phase organique est séparée et la phase aqueuse est extraite avec 3 fois 200 mL de dichlorométhane. Les phases organiques sont ensuite réunies, puis on ajoute 10 g de silice à la solution obtenue avant de la concentrer sous pression réduite : obtention d'une poudre noire que l'on utilise comme dépôt solide pour une chromatographie sur gel de silice en éluant avec un mélange de cyclohexane et d'acétate d'éthyle (9/1). On obtient 3,0 g (74%) de composé sous forme d'un solide orangé.
LC-MS : M+H = 355 (dégradation du produit, formation de l'acide boronique M+H= 273) RMN-¹H (DMSO) δ (ppm) : 1,35 (s, 12H) ; 7,0 (d, 1H) ; 7,22 (s, 1H) ; 7,55 (m, 2H) ; 8,0 (m, 3H) ; 8,1 (d, 2H) ; 8,7 (d, 1H).

### 12.2. 6-[2-(4-chlorophényl)pyrazolol[1,5-a]pyridin-5-yl]indanone

On procède suivant le mode opératoire décrit dans l'exemple 3. A partir de 0,2 g (0,56 mmol) de 2-(4-chlorophényl)-5-(4,4,5,5-tétraméthyl-[1,3,2]dioxaborolan-2-yl)pyrazolo[1,5-*a*]pyridine, obtenu à l'étape 15.1, 0,24 mg (1,14 mmol) de 6-bromo-1-indanone , de 0,55 g (1,69 mmol) de carbonate de césium, de 0,046 g (0,06 mmol) de [1,1'-bis(diphénylphosphino)ferrocène]dichloropalladium (II) et de 5 mL d'un mélange THF-eau (9/1) et après chromatographie sur gel de silice en éluant avec un mélange de cyclohexane et d'acétate d'éthyle (8/2), on obtient 0,127 g (62%) de produit attendu sous la forme d'une poudre jaune.
LC-MS : M+H = 359
RMN-¹H (DMSO) δ (ppm) : 2,72 (m, 2H) ; 3,20 (m, 2H) ; 7,15 (s, 1H) ; 7,35 (dd, 1H) ; 7,55 (m, 2H) ; 7,75 (m, 1H) ; de 8,05 à 8,20 (m, 5H) ; 8,80 (d, 1H).

### 12.3. 6-[2-(4-chlorophényl)pyrazolo[1,5-a]pyridin-5-yl]indan-1-ol (composé n°12)

A une solution de 0,12 g (0.33 mmol) de 6-[2-(4-chlorophényl)pyrazolo[1,5-*a*]pyridin-5-yl]indanone dans 16 mL d'un mélange 1/1 de méthanol et de tétrahydrofurane, refroidie à environ 5°C, sont ajoutés par portions 0,040 g (1,06 mmol) de borohydrure de sodium. Le milieu est agité 2 heures à 70°C puis refroidie à 0°C puis hydrolysé par addition goutte à goutte de 10 mL d'une solution aqueuse saturée en chlorure d'ammonium Le mileu est ensuite dilué avec 50 mL d'acétate d'éthyle et 30 mL d'eau. La phase organique est récupérée et la phase aqueuse est extraite avec 30 mL d'acétate d'éthyle. Les phases organiques sont réunies, séchées sur sulfate de sodium puis concentrées sous pression réduite. On purifie le résidu obtenu par dépôt solide en chromatographie sur gel de silice en éluant avec un mélange de cyclohexane et d'acétate d'éthyle (8/2). On obtient 0,080 g (66%) du produit attendu sous la forme d'une poudre blanche.
Point de fusion (°C) : 181-183.
LC-MS: M+H = 361
RMN-¹H (DMSO) δ (ppm) : 1,9 (m, 1H) ; 2,45 (m, 1H) ; 2,80 (m, 1H) ; 3,00 (m, 1H) ; 5,15 (m, 1H) ; 5,30 (d, 1H) ; 7,10 (s, 1H) ; 7,20 (d, 1H) ; 7,35 (d, 1H) ; 7,55 (d, 2H) ; 7,65 (d, 1H) ; 7,75 (s, 1H) ; 7,95 (s, 1H) ; 8,05 (m, 2H) ; 8,80 (d, 1H).

Les tableaux qui suivent illustrent les structures chimiques de formule générale (I) (tableau 1) et les caractéristiques physicochimiques (tableau 2) de quelques exemples de composés selon l'invention.

Dans ces tableaux :
- la colonne « Position » renseigne sur la position de substitution du groupe 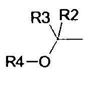 sur le noyau phényle (2, 3 ou 4) ;
- Ph signifie Phényle ;
- la colonne « PF » renseigne les points de fusion des produits en degrés Celsius (°C).

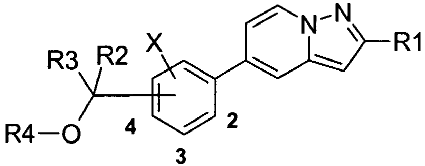

**Tableau 1**

| **N°** | **R1** | **Position** | **R2** | **R4** | **R3** | **X** |
|---|---|---|---|---|---|---|
| **1** | 4-Cl-Ph | 3 | H | H | H | H |
| **2** | 4-Cl-Ph | 3 | CH3 | H | CH3 | H |
| **3** | 4-Cl-Ph | 3 | CH3 | H | H | H |
| **4** | 4-Cl-Ph | 3 | 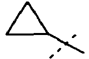 | H | H | H |
| **5** | 4-Cl-Ph | 3 | H | H | H | 2,4-(F)₂ |
| **6** | 4-F-Ph | 3 | H | H | H | H |
| **7** | 4-F-Ph | 3 | CH3 | H | CH3 | H |
| **8** | 4-F-Ph | 3 | CH3 | H | H | H |
| **9** | 4-Cl-Ph | 3 | H | CH3 | H | H |
| **10** | 4-Cl-Ph | 4 | H | H | H | H |
| **11** | 4-Cl-Ph | 2 | H | H | H | H |
| **12** | 4-Cl-Ph | 3 | H | H | -(CH₂)₂- * | |

| | | | | | | |
|---|---|---|---|---|---|---|
| *Pour le composé 12, X est en position 4. | | | | | | |

**Tableau 2**

| **N°** | **PF** | **RMN / [M+H]** |
|---|---|---|
| **1** | 161- 164°C | RMN-¹H (DMSO-d6, δ en ppm): 4,6 (d, 2H) ; 5,3 (t, 1H) ; 7,1 (s, 1H) ; 7,3 (d, 1H) ; 7,4 (d, 1H) ; 7,5 (t, 1H) ; 7,6 (d, 2H) ; 7,7 (d, 1H) ; 7,8 (s, 1H) ; 8,0 (s, 1H) ; 8,1 (d, 2H) ; 8,8 (d, 1H) ; M+H=335. |
| **2** | 159-161°C | RMN-¹H (DMSO) 8 (ppm) : 1,50 (s, 6H) ; 5,10 (s, 1H) ; 7,15 (s, 1H) ; 7,30 (d, 2H) ; 7,45 (t, 1H) ; 7,55 (m, 2H) ; 7,70 (m, 1H) ; 7,90 (s, 1H) ; 8,00 (m, 1H) ; 8,05 (m, 2H) ;8,80 (d, 1H) ; M+H= 363 |
| **3** | 93-95°C | RMN-¹H (DMSO) δ (ppm) : 1,45 (d, 3H) ; 4,85 (m, 1H) ; 5,25 (d, 1H) ; 7,15 (s, 1H) ; 7,30 (d, 1H) ; 7,45 (m, 2H) ; 7,60 (m, 2H) ; 7,70 (d, 1H) ; 7,80 (s, 1H) ; 8,05 (m, 3H) ; 8,80 (d, 1H); M+H= 349 |
| **4** | 141-143°C | RMN-¹H (DMSO) δ (ppm) : 0,45 (m, 4H) ; 1,15 (m, 1H) ; 4,10 (m, 1H) ; 5,25 (s, 1H) ; 7,15 (s, 1H) ; 7,30 (d, 1H) ; 7,50 (m, 2H) ; 7,60 (d, 2H) ; 7,70 (m, 1H) ; 7,80 (s, 1H) ; 8,00 (s, 1H) ; 8,10 (d, 2H) ; 8,80 (d, 1H) ; M+H= 375 |
| **5** | 190-192°C | RMN-¹H (DMSO) δ (ppm) : 4,60 (d, 2H) ; 5,35 (t, 1H) ; 7,10 (d, 1H) ; 7,20 (s, 1H) ; 7,25 (t, 1H) ; 7,60 (d, 2H) ; 7,70 (m, 1H) ; 7,90 (s, 1H) ; 8,05 (d, 2H) ; 8,80 (d, 1H) ; M+H= 371 |
| **6** | 141-143°C | RMN-¹H (DMSO) δ (ppm) : 4,65 (d, 2H) ; 5,30 (t, 1H) ; 7,10 (s, 1H) ; de 7,25 à 7,40 (m, 4H) ; 7,50 (m, 1H) ; 7,70 (d, 1H) ; 7,80 (s, 1H) ; 8,00 (s, 1H) ; 8,05 (t, 2H) ; 8,80 (d, 1H) ; M+H= 319 |
| **7** | 162-164°C | RMN-¹H (DMSO) δ (ppm) : 1,50 (s, 6H) ; 5,10 (s, 1H) ; 7,10 (s, 1H) ; 7,25 (d, 1H) ; 7,35 (t, 2H) ; 7,45 (t, 1H) ; 7,55 (m, 1H) ; 7,65 (m, 1H) ; 7,90 (s, 1H) ; 8,00 (s, 1H) ; 8,10 (t, 2H) ; 8,80 (d, 1H) ; M+H= 347 |
| **8** | 132-134°C | RMN-¹H (DMSO) δ (ppm): 1,42 (d, 3H) ; 4.85 (q, 1H) ; 5,25 (s, 1H) ; 7,11 (s, 1H) ; 7,25 (d, 1H) ; 7,34 (m, 2H) ; de 7,40 à 7,50 (m, 2H) ; 7,69 (d, 1H) ; 7,80 (d, 1H) ; 8,00 (s, 1H) ; 8,08 (m, 2H) ; 8,75 (d, 1H) ; M+H=333. |
| **9** | 130-132°C | RMN-¹H (DMSO) δ (ppm) : 3,35 (s, 3H) ; 4,55 (s, 2H) ; 7,15 (s, 1H) ; 7,30 (d, 1H) ; 7,40 (s, 1H) ; 7,55 (m, 3H) ; 7,80 (m, 2H) ; 8,05 (m, 3H) ; 8,80 (d, 1H); M+H= 349 |
| **10** | 214-216°C | RMN-¹H (DMSO) δ (ppm) : 4,60 (d, 2H) ; 5,30 (d, 1H) ; 7,20 (s, 1H) ; 7,30 (d, 1H) ; 7,45 (d, 2H) ; 7,60 (d, 2H) ; 7,80 (d, 2H) ; 8,05 (m, 3H) ; 8,80 (d, 1H); M+H= 335 |
| **11** | 144-146°C | RMN-¹H (DMSO) δ (ppm) : 4,50 (d, 2H) ; 5,25 (d, 1H) ; 7,00 (d, 1H) ; 7,15 (s, 1H) ; de 7,40 à 7,50 (m, 3H) ; de 7,55 à 7,65 (m, 3H) ; 7,75 (s, 1H) ; 8,05 (d, 2H) ; 8,75 (d, 1H) ; M+H= 335 |
| **12** | 181-183°C | RMN-¹H (DMSO) δ (ppm) : 1,9 (m, 1H) ; 2,45 (m, 1H) ; 2,80 (m, 1H) ; 3,00 (m, 1H) ; 5,15 (m, 1H) ; 5,30 (d, 1H) ; 7,10 (s, 1H) ; 7,20 (d, 1H) ; 7,35 (d, 1H) ; 7,55 (d, 2H) ; 7,65 (d, 1H) ; 7,75 (s, 1H) ; 7,95 (s, 1H) ; 8,05 (m, 2H) ; 8,80 (d, 1H); M+H= 361 |

Les composés selon l'invention ont fait l'objet d'essais pharmacologiques permettant de déterminer leur effet modulateur sur NOT.

### Evaluation de l'activité in vitro sur cellules N2A

L'activité des composés selon l'invention a été évaluée sur une lignée cellulaire (N2A) exprimant de manière endogène le récepteur de souris Nurr1 et transfectée de manière stable avec l'élément de réponse liant NOT (NBRE) couplé au gène rapporteur luciférase. Les essais ont été réalisés selon le mode opératoire décrit ci dessous.

La lignée cellulaire Neuro-2A provient de source commerciale standard (ATCC). Le clone Neuro-2A a été obtenu à partir d'une tumeur spontanée provenant d'une souche de souris A albino par R.J Klebe et col. Cette lignée Neuro-2A est ensuite stablement transfectée avec 8NBRE-luciférase. Les cellules N2A-8NBRE sont cultivées jusqu'à confluence dans des flacons de culture de 75 cm² contenant du DMEM supplémenté par 10% de sérum foetal de veau, 4,5 g/L de glucose et 0,4 mg/ml de Généticine. Après une semaine de culture, les cellules sont récupérées par de la trypsine 0,25% pendant 30 secondes puis remises en suspension dans du DMEM sans rouge de phénol contenant 4,5g/L de glucose, 10% de sérum délipidé Hyclone et déposées dans des plaques blanches 96 puits à fond transparent. Les cellules sont déposées à raison de 60.000 par puits dans 75 µL pendant 24 heures avant l'addition des produits. Les produits sont appliqués dans 25 µL et incubés 24 heures supplémentaires. Le jour de la mesure, on ajoute à chaque puits un volume équivalent (100µL) de Steadylite, puis on attend 30 minutes pour obtenir une lyse complète des cellules et la production maximale du signal. Les plaques sont ensuite mesurées dans un compteur de luminescence pour microplaques après avoir été scellées par un film adhésif. Les produits sont préparés sous forme de solution stock à 10⁻² M, puis dilués dans 100% de DMSO. Chaque concentration de produit est préalablement diluée dans du milieu de culture avant incubation avec les cellules contenant ainsi 0,625% final de DMSO.
Les meilleurs composés ont une CE50 comprise entre 0,1 nM et 10 µM.
Par exemple, les composés n° 1, 11 et 12 ont montré une CE50 de respectivement 0,2 ; 1,7 et 164 nM.

Il apparaît donc que les composés selon l'invention ont un effet modulateur de NOT.

Les composés de la présente invention possèdent également toutes les propriétés requises pour le développement d'un médicament, notamment un profil d'activité et de sécurité amélioré.

Les composés selon l'invention peuvent donc être utilisés pour la préparation de médicaments pour leur application en thérapeutique dans le traitement ou la prévention de maladies impliquant les récepteurs NOT.

Ainsi, selon un autre de ses aspects, l'invention a pour objet des médicaments qui comprennent un composé de formule (I), ou un sel d'addition de ce dernier à un acide pharmaceutiquement acceptable.
Ces médicaments trouvent leur emploi en thérapeutique, notamment dans le traitement et la prévention des maladies neurodégénératives telles que par exemple la maladie de Parkinson, d'Alzheimer, les tauopathies (ex. la paralysie progressive supranucléaire, la démence frontotemporale, la dégénérescence corticobasale, la maladie de Pick); les traumatismes cérébraux comme l'ischémie et les traumatismes crâniens et l'épilepsie ; les maladies psychiatriques comme la schizophrénie, la dépression, la dépendance à une substance, les troubles du déficit de l'attention et de l'hyperactivité ; les maladies inflammatoires du système nerveux central comme la sclérose en plaques, encéphalite, myélite et encéphalomyélite et autres maladies inflammatoires comme les pathologies vasculaires, l'athérosclérose, les inflammations des articulations, l'arthrose, l'arthrite rhumatoïde ; l'ostéoarthrite, la maladie de Crohn, colite ulcéreuse; les maladies inflammatoires allergiques telle que l'asthme, les maladies auto-immunes comme le diabète de type 1, lupus, sclérodermie, syndrome de Guillain-Barré, maladie d'Addison et autres maladies immuno-médiées; l'ostéoporose; les cancers.

Ces composés pourraient être aussi utilisés comme traitement associé à des greffes et/ou transplantations de cellules souches.
Selon un autre de ses aspects, la présente invention concerne des compositions pharmaceutiques comprenant, en tant que principe actif, un composé selon l'invention. Ces compositions pharmaceutiques contiennent une dose efficace d'au moins un composé selon l'invention, ou un sel pharmaceutiquement acceptable, dudit composé, ainsi qu'au moins un excipient pharmaceutiquement acceptable.

Lesdits excipients sont choisis selon la forme pharmaceutique et le mode d'administration souhaité, parmi les excipients habituels qui sont connus de l'Homme du métier.

Dans les compositions pharmaceutiques de la présente invention pour l'administration orale, sublinguale, sous-cutanée, intramusculaire, intra-veineuse, topique, locale, intratrachéale, intranasale, transdermique ou rectale, le principe actif de formule (I) ci-dessus, ou son sel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscarmellose sodique | 6,0 mg |
| Amidon de maïs | 15,0 mg |
| Hydroxypropyl-méthylcellulose | 2,25 mg |
| Stéarate de magnésium | 3,0 mg |

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

La présente invention, selon un autre de ses aspects, concerne également une méthode de traitement des pathologies ci-dessus indiquées qui comprend l'administration, à un patient, d'une dose efficace d'un composé selon l'invention, ou un de ses sels pharmaceutiquement acceptables.

## Revendications

1. Composés de formule (I) : 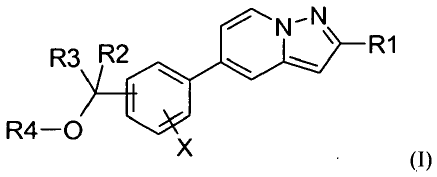 dans laquelle :
R₁ représente :
un groupe phényle ou un groupe naphthyle, ces deux groupes pouvant éventuellement être substitués par un ou plusieurs atomes ou groupes choisis indépendamment les uns des autres parmi les atomes ou groupes suivants : halogène, (C₁-C₆)alkyle, halo(C₁-C₆)alkyle, (C₁-C₆)alcoxy, halo(C₁-C₆)alcoxy, (C₁-C₆)thioalkyle, -S(O)(C₁-C₆)alkyle, -S(O)₂(C₁-C₆-alkyle), hydroxyle, hydroxy(C₁-C₆)alkylène, CHO, COOH , (C₁-C₆)alcoxy(C₁-C₆)alkylène-oxy, NRaRb, CONRaRb, SO₂NRaRb, NRcCORd, OC(O)NRaRb, OCO(C₁-C₆)alkyle, NRcC(O)ORe, NRcSO₂Re,
X représente de 1 à 4 substituants identiques ou différents l'un de l'autre choisis parmi hydrogène, halogène, (C₁-C₆)alkyle, (C₁-C₆)alcoxy, le groupe (C₁-C₆)alkyle pouvant être éventuellement substitué par un ou plusieurs atomes ou groupes choisis parmi un halogène, (C₁-C₆)alcoxy, hydroxyle,
R₂ et R₃ représentent, indépendamment l'un de l'autre,
un atome d'hydrogène,
un groupe (C₁-C₆)alkyle éventuellement substitué par un groupe Rf ;
un groupe CHO ou COOH,
X et R3 peuvent former ensemble avec les atomes de carbone qui les portent un cycle carboné de 5 à 7 atomes de carbone ;
R₄ représente un atome d'hydrogène ou un groupe (C₁-C₆)alkyle,
Ra et Rb représentent, indépendamment l'un de l'autre, un atome d'hydrogène ou un groupe (C₁-C₆)alkyle, aryl(C₁-C₆)alkylène ou aryle ;
ou Ra et Rb forment ensemble, avec l'atome d'azote qui les porte, un groupe azétidine, pyrrolidine, pipéridine, azépine, morpholine, thiomorpholine, pipérazine, homopipérazine,
ce groupe étant éventuellement substitué par un groupe (C₁-C₆)alkyle, aryle ou aryl(C₁-C₆)alkylène ;
Rc et Rd représentent, indépendamment l'un de l'autre, un atome d'hydrogène ou un groupe (C₁-C₆)alkyle, aryl(C₁-C₆)alkylène, aryle ;
ou Rc et Rd forment ensemble un groupe (C₂-C₅)alkylène ;
Re représente un groupe (C₁-C₆)alkyle, aryl(C₁-C₆)alkylène, aryle ;
ou Rc et Re forment ensemble un groupe (C₂-C₅)alkylène ;
Rf représente un groupe hydroxyle, un groupe oxo, CHO ou COOH,
à l'état de base ou de sel d'addition à un acide.

2. Composés de formule (I) selon la revendication 1, **caractérisé en ce que**
R1 représente un groupe phényle substitué par un halogène ;
R2 et R3 représentent indépendamment l'un de l'autre un atome d'hydrogène, un groupe (C₁-C₆)alkyle,
R4 représente un atome d'hydrogène ou un groupe (C₁-C₆)alkyle;
X représente un ou plusieurs atomes d'hydrogène ou d'halogène, ou X et R3 peuvent former ensemble avec les atomes de carbone qui les portent un cycle carboné de 5 atomes de carbone ;
à l'état de base ou de sel d'addition à un acide.

3. Composés de formule (I) selon la revendication 1, **caractérisé en ce que**
R1 représente un groupe phényle substitué par un chlore ou un fluor ;
R2 et R3 représentent indépendamment l'un de l'autre un atome d'hydrogène, un méthyle, ou un groupe cyclopropyle ;
R4 représente un atome d'hydrogène ou un groupe méthyle;
X représente un ou plusieurs atomes d'hydrogène ou de fluor, ou X et R3 peuvent former ensemble avec les atomes de carbone qui les portent et avec le cycle benzofusionné un groupe indane,
à l'état de base ou de sel d'addition à un acide.

4. Composés
{3-[2-(4-Chlorophényl)pyrazolo[1,5-*a*]pyridin-5-yl]phényl}méthanol
2-{3-[2-(4-Chlorophényl)pyrazolo[1,5-*a*]pyridin-5-yl]phényl}propan-2-ol
1-{3-[2-(4-Chlorophényl)pyrazolo[1,5-*a*]pyridin-5-yl]phényl}éthanol
1- {3-[2-(4-Chlorophényl)pyrazolo[1,5-*a*]pyridin-5-yl]phényl}cyclopropylmethanol
{3-[2-(4-Chlorophényl)pyrazolo[1,5-*a*]pyridin-5-yl]-2,6-difluorophényl]méthanol
{3-[2-(4-Fluorophényl)pyrazolo[1,5-*a*]pyridin-5-yl]phényl}méthanol
2-{3-[2-(4-Fluorophényl)pyrazolo[1,5-*a*]pyridin-5-yl]phényl}propan-2-ol
1-{3-[2-(4-Fluorophényl)pyrazolo[1,5-*a*]pyridin-5-yl]phényl} éthanol
2-((4-Chlorophényl)-5-(3-méthoxyméthylphényl)pyrazolo[1,5-*a*]pyridine
{4-[2-(4-Chlorophényl)pyrazolo[1,5-*a*]pyridin-5-yl]phényl}méthanol
{2-[2-(4-Chlorophényl)pyrazolo[1,5-*a*]pyridin-5-yl]phényl}méthanol
6-[2-(4-Chlorophényl)pyrazolo[1,5-*a*]pyridin-5-yl]indan-1-ol

5. Médicament, **caractérisé en ce qu'**il comprend un composé de formule (I) selon l'une quelconque des revendications 1 à 4, ou un sel d'addition de ce composé à un acide pharmaceutiquement acceptable.

6. Composition pharmaceutique, **caractérisée en ce qu'**elle comprend un composé de formule (I) selon l'une quelconque des revendications 1 à 4, ou un sel pharmaceutiquement acceptable, de ce composé, ainsi qu'au moins un excipient pharmaceutiquement acceptable.

7. Utilisation d'un composé de formule (I) selon l'une quelconque des revendications 1 à 4 pour la préparation d'un médicament destiné au traitement et à la prévention des maladies neurodégénératives.

8. Utilisation d'un composé de formule (I) selon l'une quelconque des revendications 1 à 4 pour la préparation d'un médicament destiné au traitement et à la prévention des traumatismes cérébraux et de l'épilepsie.

9. Utilisation d'un composé de formule (I) selon l'une quelconque des revendications 1 à 4 pour la préparation d'un médicament destiné au traitement et à la prévention des maladies psychiatriques.

10. Utilisation d'un composé de formule (I) selon l'une quelconque des revendications 1 à 4 pour la préparation d'un médicament destiné au traitement et à la prévention des maladies inflammatoires.

11. Utilisation d'un composé de formule (1) selon l'une quelconque des revendications 1 à 4 pour la préparation d'un médicament destiné au traitement et à la prévention de l'ostéoporose et les cancers.

12. Utilisation d'un composé de formule (I) selon l'une quelconque des revendications 1 à 4 pour la préparation d'un médicament destiné au traitement et à la prévention de la maladie de Parkinson, d'Alzheimer, des tauopathies, de la sclérose en plaques.

13. Utilisation d'un composé de formule (I) selon l'une quelconque des revendications 1 à 4 pour la préparation d'un médicament destiné au traitement et à la prévention de la schizophrénie, la dépression, la dépendance à une substance, les troubles du déficit de l'attention et de l'hyperactivité.

14. Composé de formule (IIb1)

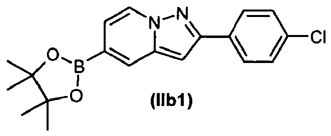

15. Utilisation du composé selon la revendication 14 pour la synthèse de produits de formule générale (I) selon la revendication 1.

## Patentansprüche

1. Verbindungen der Formel (I): 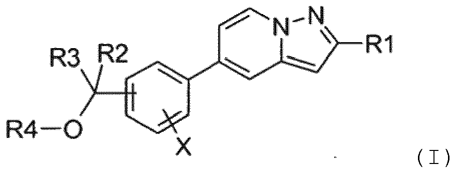 worin:
R1 für:
eine Phenylgruppe oder eine Naphthylgruppe steht, wobei diese beiden Gruppen gegebenenfalls durch ein oder mehrere Atome bzw. eine oder mehrere Gruppen substituiert sein können, die unabhängig voneinander aus den folgenden Atomen bzw. Gruppen ausgewählt sind: Halogen, (C₁-C₆)-Alkyl, Halogen-(C₁-C₆)-alkyl, (C₁-C₆)-Alkoxy, Halogen-(C₁-C₆)-alkoxy, (C₁-C₆)-Thioalkyl, -S(O)-(C₁-C₆)-Alkyl, -S(O)₂-(C₁-C₆)-Alkyl, Hydroxyl, Hydroxy-(C₁-C₆)-alkylen, CHO, COOH, (C₁-C₆)-Alkoxy-(C₁-C₆)-alkylenoxy, NRaRb, CONRaRb, SO₂NRaRb, NRcCORd, OC(O)NRaRb, OCO-(C₁-C₆)-Alkyl, NRcC(O)ORe, NRcSO₂Re,
X für 1 bis 4 gleiche oder voneinander verschiedene Substituenten steht, die aus Wasserstoff, Halogen, (C₁-C₆)-Alkyl und (C₁-C₆)-Alkoxy ausgewählt sind, wobei die (C₁-C₆)-Alkylgruppe gegebenenfalls durch ein oder mehrere Atome bzw. eine oder mehrere Gruppen, die aus Halogen, (C₁-C₆)-Alkoxy und Hydroxyl ausgewählt sind, substituiert sein können,
R₂ und R₃ unabhängig voneinander für
ein Wasserstoffatom,
eine (C₁-C₆)-Alkylgruppe, die gegebenenfalls durch eine Gruppe Rf substituiert ist,
eine CHO-oder COOH-Gruppe
stehen,
X und R3 zusammen mit den Kohlenstoffatomen, an die sie gebunden sind, einen Kohlenstoffring mit 5 bis 7 Kohlenstoffatomen bilden können;
R₄ für ein Wasserstoffatom oder eine (C₁-C₆)-Alkylgruppe steht,
Ra und Rb unabhängig voneinander für ein Wasserstoffatom oder eine (C₁-C₆)-Alkyl-, Aryl-(C₁-C₆)-alkylen- oder Arylgruppe stehen;
oder Ra und Rb zusammen mit dem Stickstoffatom, an das sie gebunden sind, eine Azetidin-, Pyrrolidin-, Piperidin-, Azepin-, Morpholin-, Thiomorpholin-, Piperazin- oder Homopiperazingruppe bilden, wobei diese Gruppe gegebenenfalls durch eine (C₁-C₆)-Alkyl-, Aryl- oder Aryl-(C₁-C₆)-alkylengruppe substituiert ist;
Rc und Rd unabhängig voneinander für ein Wasserstoffatom oder eine (C₁-C₆)-Alkyl-, Aryl-(C₁-C₆)-alkylen- oder Arylgruppe stehen;
oder Rc und Rd zusammen eine (C₂-C₅) -Alkylengruppe bilden;
Re für eine (C₁-C₆)-Alkyl-, Aryl-(C₁-C₆)-alkylen- oder Arylgruppe steht;
oder Rc und Re zusammen eine (C₂-C₅) -Alkylengruppe bilden;
Rf für eine Hydroxylgruppe oder eine Oxo-, CHO-oder COOH-Gruppe steht,
in Basen- oder Säureadditionssalzform.

2. Verbindungen der Formel (I) nach Anspruch 1, **dadurch gekennzeichnet, dass**
R1 für eine Phenylgruppe, die durch ein Halogen substituiert ist, steht;
R2 und R3 unabhängig voneinander für ein Wasserstoffatom oder eine (C₁-C₆)-Alkylgruppe stehen,
R4 für ein Wasserstoffatom oder eine (C₁-C₆)-Alkylgruppe steht;
X für ein oder mehrere Wasserstoff- oder Halogenatome steht oder X und R3 zusammen mit den Kohlenstoffatomen, an die sie gebunden sind, einen Kohlenstoffring mit 5 Kohlenstoffatomen bilden können;
in Basen- oder Säureadditionssalzform.

3. Verbindungen der Formel (I) nach Anspruch 1, **dadurch gekennzeichnet, dass**
R1 für eine Phenylgruppe, die durch ein Chlor oder ein Fluor substituiert ist, steht;
R2 und R3 unabhängig voneinander für ein Wasserstoffatom, ein Methyl oder eine Cyclopropylgruppe stehen,
R4 für ein Wasserstoffatom oder eine Methylgruppe steht;
X für ein oder mehrere Wasserstoff- oder Fluoratome steht oder X und R3 zusammen mit den Kohlenstoffatomen, an die sie gebunden sind, und mit dem benzoanellierten Ring eine Indangruppe bilden können;
in Basen- oder Säureadditionssalzform.

4. Verbindungen
{3-[2-(4-Chlorphenyl)pyrazolo[1,5-*a*]pyridin-5-yl]phenyl}methanol
2-{3-[2-(4-Chlorphenyl)pyrazolo[1,5-*a*]pyridin-5-yl]phenyl}propan-2-ol
1-{3-[2-(4-Chlorphenyl)pyrazolo[1,5-*a*]pyridin-5-yl]phenyl}ethanol
1-{3-[2-(4-Chlorphenyl)pyrazolo[1,5-*a*]pyridin-5-yl]phenyl}cyclopropylmethanol
{3-[2-(4-Chlorphenyl)pyrazolo[1,5-*a*]pyridin-5-yl]-2,6-difluorphenyl}methanol
{3-[2-(4-Fluorphenyl)pyrazolo[1,5-*a*]pyridin-5-yl]phenyl}methanol
2-{3-[2-(4-Fluorphenyl)pyrazolo[1,5-*a*]pyridin-5-yl]phenyl}propan-2-ol
1-{3-[2-(4-Fluorphenyl)pyrazolo[1,5-*a*]pyridin-5-yl]phenyl}ethanol
2-(4-Chlorphenyl)-5-(3-methoxymethylphenyl)-pyrazolo[1,5-a]pyridin
{4-[2-(4-Chlorphenyl)pyrazolo[1,5-*a*]pyridin-5-yl]phenyl}methanol
{2-[2-(4-Chlorphenyl)pyrazolo[1,5-*a*]pyridin-5-yl]phenyl}methanol
6-[2-(4-Chlorphenyl)pyrazolo[1,5-a]pyridin-5-yl]indan-1-ol.

5. Medikament, **dadurch gekennzeichnet, dass** es eine Verbindung der Formel (I) nach einem der Ansprüche 1 bis 4 oder ein Additionssalz dieser Verbindung mit einer pharmazeutisch unbedenklichen Säure umfasst.

6. Pharmazeutische Zusammensetzung, **dadurch gekennzeichnet, daß** sie eine Verbindung der Formel (I) nach einem der Ansprüche 1 bis 4 oder ein pharmazeutisch unbedenkliches Salz dieser Verbindung sowie mindestens einen pharmazeutisch unbedenklichen Hilfsstoff enthält.

7. Verwendung einer Verbindung der Formel (I) nach einem der Ansprüche 1 bis 4 zur Herstellung eines Medikaments zur Behandlung und Prävention von neurodegenerativen Erkrankungen.

8. Verwendung einer Verbindung der Formel (I) nach einem der Ansprüche 1 bis 4 zur Herstellung eines Medikaments zur Behandlung und Prävention von Hirntraumen und Epilepsie.

9. Verwendung einer Verbindung der Formel (I) nach einem der Ansprüche 1 bis 4 zur Herstellung eines Medikaments zur Behandlung und Prävention von psychiatrischen Erkrankungen.

10. Verwendung einer Verbindung der Formel (I) nach einem der Ansprüche 1 bis 4 zur Herstellung eines Medikaments zur Behandlung und Prävention von entzündlichen Erkrankungen.

11. Verwendung einer Verbindung der Formel (I) nach einem der Ansprüche 1 bis 4 zur Herstellung eines Medikaments zur Behandlung und Prävention von Osteoporose und Krebserkrankungen.

12. Verwendung einer Verbindung der Formel (I) nach einem der Ansprüche 1 bis 4 zur Herstellung eines Medikaments zur Behandlung und Prävention von Parkinson-Krankheit, Alzheimer-Krankheit, Tauopathien und multipler Sklerose.

13. Verwendung einer Verbindung der Formel (I) nach einem der Ansprüche 1 bis 4 zur Herstellung eines Medikaments zur Behandlung und Prävention von Schizophrenie, Depression, Substanzabhängigkeit und Aufmerksamkeitsdefizit-Hyperaktivitätssyndrom.

14. Verbindung der Formel (IIb1)

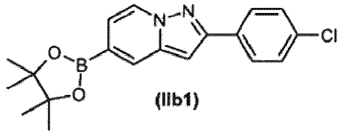

15. Verwendung der Verbindungen nach Anspruch 14 zur Synthese von Produkten der allgemeinen Formel (I) nach Anspruch 1.

## Claims

1. Compounds of formula (I): 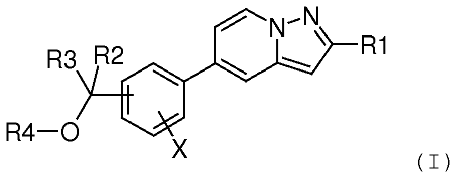 in which:
R1 represents:
a phenyl group or a naphthyl group, it being possible for these two groups optionally to be substituted by one or more atoms or groups chosen, independently of one another, from the following atoms or groups: halogen, (C₁-C₆)alkyl, halo(C₁-C₆)alkyl, (C₁-C₆)alkoxy, halo (C₁-C₆) alkoxy, (C₁-C₆) thioalkyl, -S(O)(C₁-C₆)alkyl, -S(O)₂(C₁-C₆)alkyl, hydroxyl, hydroxy(C₁-C₆)alkylene, CHO, COOH, (C₁-C₆)alkoxy(C₁-C₆)alkyleneoxy, NRaRb, CONRaRb, SO₂NRaRb, NRcCORd, OC(O)NRaRb, OCO(C₁-C₆)alkyl, NRcC(O)ORe or NRcSO₂Re,
X represents from 1 to 4 substituents which are identical to or different from one another and which are chosen from hydrogen, halogen, (C₁-C₆)alkyl or (C₁-C₆)alkoxy, it being possible for the (C₁-C₆)alkyl group to be optionally substituted by one or more atoms or groups chosen from a halogen, (C₁-C₆)alkoxy or hydroxyl;
R2 and R3 represent, independently of one another,
a hydrogen atom,
a (C₁-C₆)alkyl group optionally substituted by an Rf group;
a CHO or COOH group,
X and R3 can together form, with the carbon atoms which carry them, a carbocycle of 5 to 7 carbon atoms;
R4 represents a hydrogen atom or a (C₁-C₆)alkyl group,
Ra and Rb represent, independently of one another, a hydrogen atom or a (C₁-C₆)alkyl, aryl(C₁-C₆)alkylene or aryl group;
or Ra and Rb together form, with the nitrogen atom which carries them, an azetidine, pyrrolidine, piperidine, azepine, morpholine, thiomorpholine, piperazine or homopiperazine group, this group being optionally substituted by a (C₁-C₆)alkyl, aryl or aryl(C₁-C₆)alkylene group;
Rc and Rd represent, independently of one another, a hydrogen atom or a (C₁-C₆)alkyl, aryl(C₁-C₆)alkylene or aryl group;
or Rc and Rd together form a (C₂-C₅)alkylene group;
Re represents a (C₁-C₆) alkyl, aryl(C₁-C₆)alkylene or aryl group;
or Rc and Re together form a (C₂-C₅)alkylene group;
Rf represents a hydroxyl, oxo, CHO or COOH group,
in the form of the base or of an addition salt with an acid.

2. Compounds of formula (I) according to Claim 1, **characterized in that**
R1 represents a phenyl group substituted by a halogen;
R2 and R3 represent, independently of one another, a hydrogen atom or a (C₁-C₆)alkyl group,
R4 represents a hydrogen atom or a (C₁-C₆)alkyl group;
X represents one or more hydrogen or halogen atoms, or X and R3 can form, together with the carbon atoms which carry them, a carbocycle of 5 carbon atoms;
in the form of the base or of an addition salt with an acid.

3. Compounds of formula (I) according to Claim 1, **characterized in that**
R1 represents a phenyl group substituted by a chlorine or a fluorine;
R2 and R3 represent, independently of one another, a hydrogen atom, a methyl or a cyclopropyl group;
R4 represents a hydrogen atom or a methyl group;
X represents one or more hydrogen or fluorine atoms, or X and R3 can form, together with the carbon atoms which carry them and with the benzofused ring, an indane group,
in the form of the base or of an addition salt with an acid.

4. Compounds
{3-[2-(4-Chlorophenyl)pyrazolo[1,5-a]pyridin-5-yl]phenyl}methanol
2-{3-[2-(4-Chlorophenyl)pyrazolo[1,5-a]pyridin-5-yl]phenyl}propan-2-ol
1-{3-[2-(4-Chlorophenyl)pyrazolo[1,5-a]pyridin-5-yl]phenyl}ethanol
1-{3-[2-(4-Chlorophenyl)pyrazolo[1,5-a]pyridin-5-yl]phenyl}cyclopropylmethanol
{3-[2-(4-Chlorophenyl)pyrazolo[1,5-a]pyridin-5-yl]-2,6-difluorophenyl}methanol
{3-[2-(4-Fluorophenyl)pyrazolo[1,5-a]pyridin-5-yl]phenyl}methanol
2-{3-[2-(4-Fluorophenyl)pyrazolo[1,5-a]pyridin-5-yl]phenyl}propan-2-ol
1-{3-[2-(4-Fluorophenyl)pyrazolo[1,5-a]pyridin-5-yl]phenyl}ethanol
2-(4-Chlorophenyl)-5-(3-methoxymethylphenyl)pyrazolo[1,5-a]pyridine
{4-[2-(4-Chlorophenyl)pyrazolo[1,5-a]pyridin-5-yl]phenyl}methanol
{2-[2-(4-Chlorophenyl)pyrazolo[1,5-a]pyridin-5-yl]phenyl}methanol
6-[2-(4-Chlorophenyl)pyrazolo[1,5-a]pyridin-5-yl]indan-1-ol

5. Medicament, **characterized in that** it comprises a compound of formula (I) according to any one of Claims 1 to 4 or an addition salt of this compound with a pharmaceutically acceptable acid.

6. Pharmaceutical composition, **characterized in that** it comprises a compound of formula (I) according to any one of Claims 1 to 4, or a pharmaceutically acceptable salt of this compound, and at least one pharmaceutically acceptable excipient.

7. Use of a compound of formula (I) according to any one of Claims 1 to 4 in the preparation of a medicament intended for the treatment and prevention of neurodegenerative diseases.

8. Use of a compound of formula (I) according to any one of Claims 1 to 4 in the preparation of a medicament intended for the treatment and prevention of cerebral traumas and epilepsy.

9. Use of a compound of formula (I) according to any one of Claims 1 to 4 in the preparation of a medicament intended for the treatment and prevention of psychiatric diseases.

10. Use of a compound of formula (I) according to any one of Claims 1 to 4 in the preparation of a medicament intended for the treatment and prevention of inflammatory diseases.

11. Use of a compound of formula (I) according to any one of Claims 1 to 4 in the preparation of a medicament intended for the treatment and prevention of osteoporosis and cancers.

12. Use of a compound of formula (I) according to any one of Claims 1 to 4 in the preparation of a medicament intended for the treatment and prevention of Parkinson's disease, Alzheimer's disease, tauopathies or multiple sclerosis.

13. Use of a compound of formula (I) according to any one of Claims 1 to 4 in the preparation of a medicament intended for the treatment and prevention of schizophrenia, depression, substance dependence or attention deficit and hyperactivity disorders.

14. Compound of formula (IIb1)

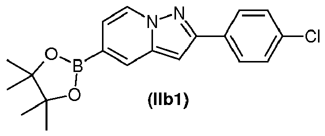

15. Use of the compound according to Claim 14 in the synthesis of products of general formula (I) according to Claim 1.
